# EUROPEAN PATENT APPLICATION

(11) **EP 4 249 479 A1**
(43) Date of publication of application: **27.09.2023**
(21) Application number: 21893992.4
(22) Date of filing: 19.11.2021
(51) Int. Cl.: C07D 309/10, C07D 309/02, C07D 335/02, C07D 405/12, C07D 407/10, C07D 409/10, C07D 409/12, A61K 31/7034, A61K 31/351, A61K 31/381, A61K 31/382, A61K 31/506, A61P 3/10

(54) **GLUCOSIDE DERIVATIVE, AND PREPARATION METHOD THEREFOR AND APPLICATION THEREOF**

(30) Priority: 19.11.2020 CN 202011302722
(71) Applicant: Beijing Increase Innovative Drug Co., Ltd., Beijing 102200 (CN)
(72) Inventor: ZHANG, Hongwu, Beijing 102200 (CN); HU, Jie, Beijing 102200 (CN); XUE, Chunmei, Beijing 102200 (CN); HU, Jing, Beijing 102200 (CN); JU, Zhihe, Beijing 102200 (CN)
(74) Representative: ABG Intellectual Property Law, S.L.
(86) International application number: PCT/CN2021/131604
(87) International publication number: WO 2022/105845

(57) **Abstract**

A glucoside derivative, and a preparation method therefor and an application thereof. Specifically, provided is a glucoside derivative, which is a compound as represented by formula (I) or a pharmaceutically acceptable salt thereof. The glucoside derivative can be used for treatment of diabetes mellitus II or diabetes mellitus I.

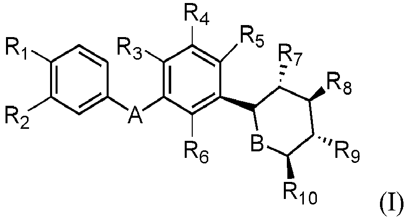

## Description

This application claims the priority of Chinese Patent Application No. 202011302722.4, filed with the China National Intellectual Property Administration on November 19, 2020, and titled with "GLUCOSIDE DERIVATIVE, AND PREPARATION METHOD THEREFOR AND APPLICATION THEREOF", which is hereby incorporated by reference in its entirety.

### FIELD

The present disclosure relates to the technical field of chemical medicines, in particular to a glycoside derivative, a preparation method thereof and use thereof.

### BACKGROUND

At present, there are more than 400 million people with diabetes mellitus in the world, including about 110 million people with diabetes mellitus in China. Therefore, drugs for treating diabetes mellitus have huge market value. Diabetes mellitus is a chronic comprehensive disease mainly characterized by the disorder of glucose metabolism caused by absolute or relative deficiency of insulin, or the decrease in sensitivity of target cells to insulin. The occurrence of type 2 (type II) diabetes mellitus is the result of the combined effect of peripheral insulin resistance and β-cell dysfunction.

The design concept of the present patent comes from the traditional Chinese medicine mango leaf. Mango leaves are used folkly for lowering blood glucose. Modern pharmacological studies have shown that the active ingredient of mango leaves for lowering blood glucose is the natural product mangiferin. The compound of the present patent is obtained through two rounds of structure optimization with mangiferin as the lead compound.

It has been reported in the literature that mangiferin exerts a direct hypoglycemic effect by improving the regeneration ability of insulin cells, insulin levels, insulin sensitivity, and glucose utilization; the intraperitoneal injection of mangiferin can significantly reduce the level of glycated hemoglobin caused by oxidative damage in diabetic rats; mangiferin has a certain therapeutic effect on diabetic nephropathy, and mangiferin can delay cardiovascular disease under oxidative stress caused by diabetes.

Mangiferin has disadvantages of low water solubility, low oral absorption bioavailability, and mild hypoglycemic effect. The structure of mangiferin is optimized to improve the pharmacokinetic properties of mangiferin and increase its bioavailability, thereby enhancing the efficacy in lowering blood glucose and reducing the risk of diabetic complications, and reducing its toxic and side effects on the human body.

Mangiferin has a similar chemical structure to that of the hypoglycemic drug SGLT-2 inhibitors. Therefore, SGLT-2 is taken as the efficacy target for the optimization of the chemical structure of mangiferin. A series of compounds with good hypoglycemic activity are obtained through the *in vitro* screening for SGLT-2 target inhibitory activity, and are further confirmed to be effective for both type 1 and type 2 diabetes animal models by animal experiments *in vivo,* which have a statistically significant advantage compared with dapagliflozin.

SGLT-2 inhibitors have the advantage of cardiovascular benefits compared with other hypoglycemic drugs. However, the SGLT-2 inhibitors in the prior art are still unsatisfactory in terms of biological activity, duration of drug effect, safety and the like.

### SUMMARY

In view of this, an object of the present invention is to provide an SGLT-2 inhibitor compound with better therapeutic effect, better safety, longer duration of drug effect, and fewer times of administration.

In order to solve the above technical problems, the present invention provides a glycoside derivative, which is a compound represented by formula I or a pharmaceutically acceptable salt thereof: 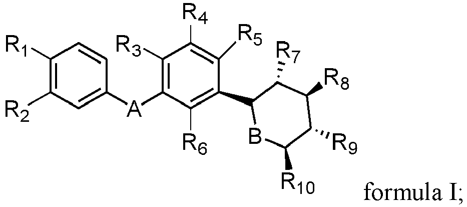
wherein, A is an oxygen atom, -(CH₂)ₘ- or -NH-; m is 1, 2 or 3;
B is an oxygen atom or a sulfur atom;
R₁, R₂, R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
alternatively, R₁ and R₂ together form heterocyclyl, cycloalkyl, aryl or heteroaryl fused to a benzene ring, and/or R₃ and R₄ together form cyclopentyl or oxacyclopentyl fused to a benzene ring;
R₇, R₈, R₉, and R₁₀ are independently selected from the group consisting of hydrogen, hydroxyl, alkyl, alkoxy and alkylthio; and
R₁₁ and R₁₁ₐ are independently selected from the group consisting of a hydrogen atom and alkyl;
wherein the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio are unsubstituted or further substituted by one or more substituents selected from the group consisting of halogen, hydroxyl, amino, carboxyl, cyano, alkyl, alkoxy and nitro.

Further preferably, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio may be further substituted by one or more substituents selected from the group consisting of halogen, hydroxyl, amino, carboxyl, cyano, C1∼C6 alkyl, C1∼C6 alkoxy and nitro.

Preferably in the present invention, A is an oxygen atom or -CH₂-.

Preferably in the present invention, B is an oxygen atom.

Preferably in the present invention, R₁, R₂, R₃, R₄, R₅ and R₆ are independently selected from the group consisting of hydrogen, hydroxyl, carboxyl, halogen, -CN, C1-C6 alkyl, C1-C6 alkoxy, C2-C16 alkoxyalkoxy, C3-C6 cycloalkyl, C6-C12 aryl, C2-C12 heteroaryl, -O-C6∼C12 aryl, -O-C2∼C12 heteroaryl, -OCH₂-C6~C12 aryl, -OCH₂-C2~C12 heteroaryl, -O-C2∼C6 heterocyclyl, -OCH₂-C2∼C6 heterocyclyl, C1-C6 ester, -NR₁₁R₁₁ₐ and 3-14 membered heterocyclyl containing 1-4 heteroatoms selected from the group consisting of N, O, S, SO and SO₂.

Wherein, R₁₁ and R₁₁ₐ are independently selected from the group consisting of a hydrogen atom and alkyl, preferably a hydrogen atom and C1~C6 alkyl. In some specific embodiments of the present invention, Rn and R₁₁ₐ are independently selected from the group consisting of H, methyl, ethyl, n-propyl, isopropyl, n-butyl, isobutyl, tert-butyl, n-pentyl, isopentyl, n-hexyl and isohexyl.

Further preferably, R₁, R₂, R₃, R₄, R₅ and R₆ are independently selected from the group consisting of hydrogen, hydroxyl, carboxyl, fluorine, chlorine, bromine, -CN, methyl, ethyl, n-propyl, isopropyl, n-butyl, isobutyl, tert-butyl, n-pentyl, isopentyl, n-hexyl, isohexyl, methoxy, ethoxy, n-propyloxy, isopropyloxy, n-butyloxy, isobutyloxy, tert-butyloxy, n-pentyloxy, isopentyloxy, n-hexyloxy, methoxymethoxy, ethoxymethoxy, ethoxyethoxy, n-propoxymethoxy, isopropoxymethoxy, n-propoxyethoxy, isopropoxyethoxy, cyclopropyl, methylcyclopropyl, cyclobutyl, cyclopentyl, cyclohexyl, phenyl, pyridyl, pyrimidyl, pyrazinyl, pyrrolyl, thienyl, furyl, oxazolyl, thiazolyl, imidazolyl, triazolyl, phenoxy, pyridyloxy, pyrimidinyloxy, pyrrolyloxy, furyloxy, thienyloxy, oxazolyloxy, benzyloxy, -OCH₂-pyridyl, -OCH₂-pyrimidinyl, -OCH₂-pyrrolyl, -OCH₂-thienyl, -OCH₂-furyl, -OCH₂-oxazolyl, -OCH₂-thiazolyl, -OCH₂-imidazolyl, -OCH₂-triazolyl, methyl ester, ethyl ester, isopropyl ester, sulfonate, amino, hexahydropyridyl, hexahydropyrazinyl, morpholinyl, tetrahydropyrrolyl and tetrahydrooxazolyl.

In the present invention, one or more hydrogen atoms of the above groups may be further substituted by one or more substituents selected from the group consisting of halogen, hydroxyl, amino, carboxyl, cyano, C1-C6 alkyl, C1-C6 alkoxy and nitro.

Further preferably, the substituent is selected from the group consisting of fluorine, chlorine, bromine, hydroxyl, amino, carboxyl, cyano, methyl, ethyl, propyl, isopropyl, methoxy, ethoxy, propoxy, isopropoxy and nitro.

In some specific embodiments of the present invention, the above phenoxy is specifically 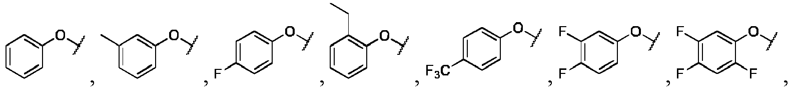 or 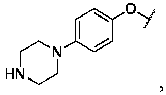 etc.

In some specific embodiments of the present invention, the above -O-C2~C12 heteroaryl is specifically 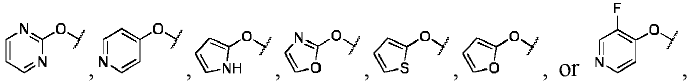 etc.

In some specific embodiments of the present invention, the above -OCH₂-C6∼C12 aryl is specifically 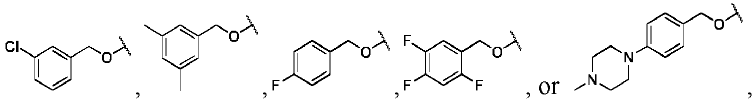 etc.

In some specific embodiments of the present invention, the above -OCH₂-C2∼C12 heteroaryl is specifically 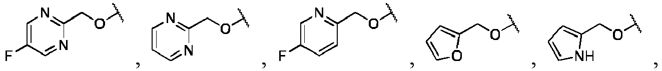 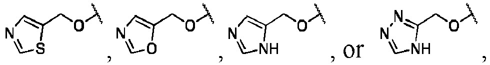 etc.

In some specific embodiments of the present invention, the above C6-C12 aryl is specifically phenyl, p-methylphenyl, p-fluorophenyl, o-chlorophenyl, m-methoxyphenyl, or 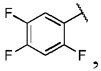 etc.

In some specific embodiments of the present invention, the above C2~C12 heteroaryl is specifically 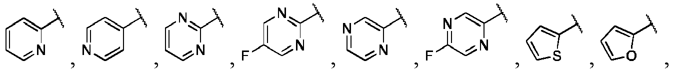 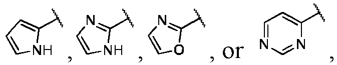 etc.

In some specific embodiments of the present invention, the above C1~C6 alkyl is specifically -CF₃, CHF₂, or CH₂F, etc.

In some specific embodiments of the present invention, the above C1~C6 alkoxy is specifically -OCHF₂, or -OCF₃, etc.

In some specific embodiments of the present invention, the above C2-C16 alkoxyalkoxy is specifically methoxymethoxy, ethoxymethoxy, ethoxyethoxy, n-propoxymethoxy, isopropoxymethoxy, n-propoxyethoxy, isopropoxyethoxy, etc.

In some specific embodiments of the present invention, the above -O-C2∼C6 heterocyclyl is specifically 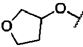 , etc.

In some specific embodiments of the present invention, the above C1~C6 ester is specifically 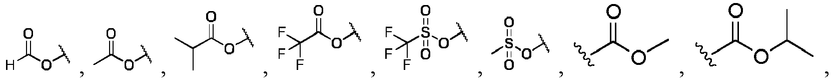 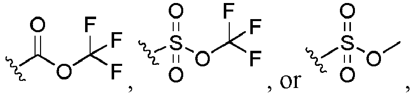 etc.

In some specific embodiments of the present invention, the above -NR₁₁R₁₁ₐ is specifically amino, 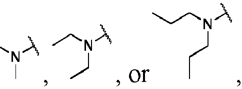 etc.

In some specific embodiments of the present invention, the above 3-14 membered heterocyclyl containing 1-4 heteroatoms selected from the group consisting of N, O, S, SO and SO₂ is specifically 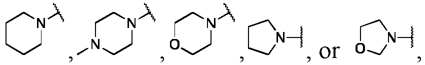 etc.

In some specific embodiments of the present invention, R₁ and R₂ together form 3-14 membered heterocyclyl containing 1-4 heteroatoms selected from the group consisting of N, O, S, SO and SO₂, C3-C6 cycloalkyl, C6-C12 aryl or C2-C12 heteroaryl fused to a benzene ring.

Preferably in the present invention, R₁ and R₂ together form cyclohexyl, phenyl, pyridyl, pyrimidyl, pyrazinyl, pyrrolyl, thienyl, furyl, oxazolyl, thiazolyl, imidazolyl, triazolyl, hexahydropyridyl, hexahydropyrazinyl, morpholinyl, tetrahydropyrrolyl, tetrahydrooxazolyl or dioxane fused to a benzene ring.

The dioxane is preferably 1,4-dioxane, i.e. dioxanyl.

Preferably in the present invention, R₇, R₈, R₉ and R₁₀ are independently selected from the group consisting of hydrogen, hydroxyl, C1-C6 alkyl, C1-C6 alkoxy and C1~C6 alkylthio.

Further preferably, R₇, R₈, R₉ and R₁₀ are independently selected from the group consisting of hydrogen, hydroxyl, methyl, ethyl, n-propyl, isopropyl, n-butyl, isobutyl, tert-butyl, n-pentyl, isopentyl, n-hexyl, isohexyl, hydroxymethyl, hydroxyethyl, methoxy, ethoxy, n-propyloxy, isopropyloxy, n-butyloxy, isobutyloxy, tert-butyloxy, n-pentyloxy, isopentyloxy, n-hexyloxy, methylthio, ethylthio, n-propylthio, isopropylthio, n-butylthio, isobutylthio, tert-butylthio, n-pentylthio, isopentylthio and n-hexylthio.

In some specific embodiments of the present invention, the glycoside derivative is a compound represented by formula I-a or a pharmaceutically acceptable salt thereof: 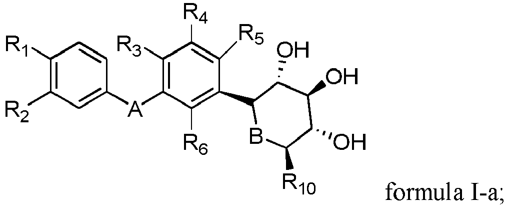 wherein, R₁, R₂, R₃, R₄, R₅, R₆, and R₁₀ are defined as the same as above, and will not be repeated here.

Preferably, R₁₀ is hydroxymethyl or methylthio.

In some specific embodiments of the present invention, the glycoside derivative is a compound represented by formula II or a pharmaceutically acceptable salt thereof: 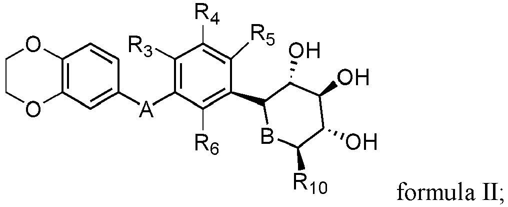
wherein, A is an oxygen atom, -(CH₂)ₘ- or -NH-; m is 1, 2 or 3;
B is an oxygen atom or a sulfur atom;
R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
R₁₀ is selected from the group consisting of hydrogen, hydroxyl, alkyl, alkoxy and alkylthio; and
R₁₁ and R₁₁ₐ are independently selected from the group consisting of a hydrogen atom and alkyl;
wherein, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio may be further substituted by one or more substituents selected from the group consisting of halogen, hydroxyl, amino, carboxyl, cyano, alkyl, alkoxy and nitro.

Further preferably, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio may be further substituted by one or more substituents selected from the group consisting of halogen, hydroxyl, amino, carboxyl, cyano, C1-C6 alkyl, C1-C6 alkoxy and nitro.

Preferably in the present invention, A is an oxygen atom or -CH₂-.

Preferably in the present invention, B is an oxygen atom.

Preferably in the present invention, R₃, R₄, Rs and R₆ are independently selected from the group consisting of hydrogen, hydroxyl, carboxyl, halogen, -CN, C1-C6 alkyl, C1-C6 alkoxy, C2-C16 alkoxyalkoxy, C3-C6 cycloalkyl, C6-C12 aryl, C2-C12 heteroaryl, -O-C6∼C12 aryl, -O-C2∼C12 heteroaryl, -OCH₂-C6~C12 aryl, -OCH₂-C2~C12 heteroaryl, -O-C2∼C6 heterocyclyl, -OCH₂-C2~C6 heterocyclyl, C1-C6 ester, -NR₁₁R₁₁ₐ and 3-14 membered heterocyclyl containing 1-4 heteroatoms selected from the group consisting of N, O, S, SO and SO₂.

Wherein, R₁₁ and R₁₁ₐ are independently selected from the group consisting of a hydrogen atom and alkyl, more preferably a hydrogen atom and C1~C6 alkyl. In some specific embodiments of the present invention, Rn and R₁₁ₐ are independently selected from the group consisting of H, methyl, ethyl, n-propyl, isopropyl, n-butyl, isobutyl, tert-butyl, n-pentyl, isopentyl, n-hexyl and isohexyl.

Further preferably, R₃, R₄, R₅ and R₆ are independently selected from the group consisting of hydrogen, hydroxyl, carboxyl, halogen, -CN, C1-C6 alkyl, C1∼C6 alkoxy and C2̂∼C16 alkoxyalkoxy.

Further preferably, R₃ is selected from the group consisting of C1∼C3 alkyl and C1~C3 alkoxy; and
R₄, Rs and R₆ are hydrogen.

The C1-C3 alkyl and C1~C3 alkoxy may be further substituted by one or more substituents selected from the group consisting of halogen, hydroxyl, amino, carboxyl, cyano and nitro.

Further preferably, R₃, R₄, R₅ and R₆ are independently selected from the group consisting of hydrogen, hydroxyl, carboxyl, fluorine, chlorine, bromine, -CN, methyl, ethyl, n-propyl, isopropyl, n-butyl, isobutyl, tert-butyl, n-pentyl, isopentyl, n-hexyl, isohexyl, methoxy, ethoxy, n-propyloxy, isopropyloxy, n-butyloxy, isobutyloxy, tert-butyloxy, n-pentyloxy, isopentyloxy, n-hexyloxy, methoxymethoxy, ethoxymethoxy, ethoxyethoxy, n-propoxymethoxy, isopropoxymethoxy, n-propoxyethoxy, isopropoxyethoxy, cyclopropyl, methylcyclopropyl, cyclobutyl, cyclopentyl, cyclohexyl, phenyl, pyridyl, pyrimidyl, pyrazinyl, pyrrolyl, thienyl, furyl, oxazolyl, thiazolyl, imidazolyl, triazolyl, phenoxy, pyridyloxy, pyrimidinyloxy, pyrrolyloxy, furyloxy, thienyloxy, oxazolyloxy, benzyloxy, -OCH₂-pyridyl, -OCH₂-pyrimidinyl, -OCH₂-pyrrolyl, -OCH₂-thienyl, -OCH₂-furyl, -OCH₂-oxazolyl, -OCH₂-thiazolyl, -OCH₂-imidazolyl, -OCH₂-triazolyl, methyl ester, ethyl ester, isopropyl ester, sulfonate, amino, hexahydropyridyl, hexahydropyrazinyl, morpholinyl, tetrahydropyrrolyl and tetrahydrooxazolyl.

In the present invention, one or more hydrogen atoms of the above groups may be further substituted by one or more substituents selected from the group consisting of halogen, hydroxyl, amino, carboxyl, cyano, C1-C6 alkyl, C1-C6 alkoxy and nitro.

Further preferably, the substituent is selected from the group consisting of fluorine, chlorine, bromine, hydroxyl, amino, carboxyl, cyano, methyl, ethyl, propyl, isopropyl, methoxy, ethoxy, propoxy, isopropoxy and nitro.

In some embodiments of the present invention:
A is -(CH₂)- or an oxygen atom;
B is an oxygen atom;
Ri and R₂ together form 5 to 6-membered heterocyclyl fused to a benzene ring or form cyclobutanyl fused to a benzene ring, wherein the 5 to 6-membered heterocyclyl contains 1 or 2 heteroatoms selected from the group consisting of O and S;
R₃ is selected from the group consisting of CH₃(CH₂)ₙ-, CH₃(CH₂)ₙO- and halogen, wherein n is selected from 0, 1, 2 and 3;
R₄ is hydrogen;
alternatively, R₃ and R₄ together form cyclopentyl or oxacyclopentyl fused to a benzene ring;
R₅ and R₆ are hydrogen;
R₇, R₈ and R₉ are hydroxyl; and

R₁₀ is selected from the group consisting of hydroxyalkyl, alkoxy and alkylthio.

In some other embodiments of the present invention:
A is -(CH₂)- or an oxygen atom;
B is an oxygen atom;
R₁ and R₂ together form 5 to 6-membered heterocyclyl fused to a benzene ring or form cyclobutanyl fused to a benzene ring, wherein the 5 to 6-membered heterocyclyl contains 1 or 2 oxygen atoms;
R₃ is selected from the group consisting of CH₃(CH₂)ₙ-, CH₃(CH₂)ₙO- and halogen, wherein n is selected from 0, 1, 2 and 3;
R₄ is hydrogen;
alternatively, R₃ and R₄ together form cyclopentyl or oxacyclopentyl fused to a benzene ring;
R₅ and R₆ are hydrogen;
R₇, R₈ and R₉ are hydroxyl; and

R₁₀ is selected from the group consisting of hydroxymethyl, methoxy and methylthio.

In some embodiments of the present invention:
A is an oxygen atom;
B is an oxygen atom;
R₁ is C₁-C₃ alkoxy;
R₂ is hydrogen;
R₃ is halogen, preferably fluorine, chlorine or bromine;
R₄ is hydrogen;
R₅ and R₆ are hydrogen;
R₇, R₈ and R₉ are hydroxyl; and
R₁₀ is hydroxyalkyl, preferably hydroxymethyl.

In some specific embodiments of the present invention, the above phenoxy is specifically 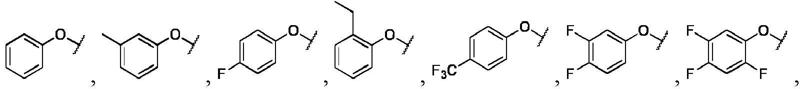 or 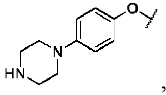 etc.

In some specific embodiments of the present invention, the above -O-C2~C12 heteroaryl is specifically 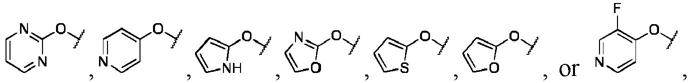 etc.

In some specific embodiments of the present invention, the above -OCH₂-C6∼C12 aryl is specifically selected from the group consisting of PhCH₂O-, 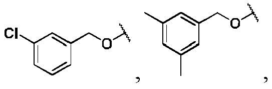 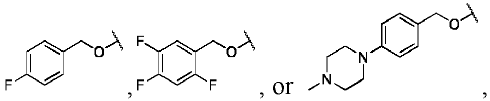 etc.

In some specific embodiments of the present invention, the above -OCH₂-C2∼C12 heteroaryl is specifically 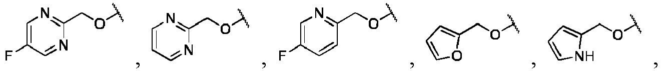 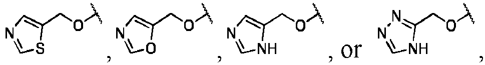 etc.

In some specific embodiments of the present invention, the above C6∼C12 aryl is specifically phenyl, p-methylphenyl, p-fluorophenyl, o-chlorophenyl, m-methoxyphenyl, or 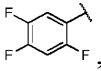 etc.

In some specific embodiments of the present invention, the above C2~C12 heteroaryl is specifically 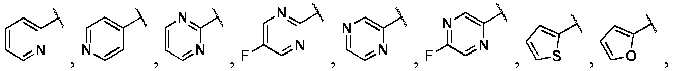 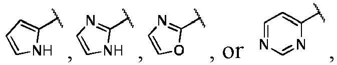 etc.

In some specific embodiments of the present invention, the above C1~C6 alkyl is specifically -CF₃, CHF₂, or CH₂F, etc.

In some specific embodiments of the present invention, the above C1~C6 alkoxy is specifically -OCHF₂, or -OCF₃, etc.

In some specific embodiments of the present invention, the above C2∼C16 alkoxyalkoxy is specifically selected from the group consisting of methoxymethoxy, ethoxymethoxy, ethoxyethoxy, n-propoxymethoxy, isopropoxymethoxy, n-propoxyethoxy, or isopropoxyethoxy, etc.

In some specific embodiments of the present invention, the above -O-C2∼C6 heterocyclyl is specifically 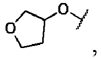 etc.

In some specific embodiments of the present invention, the above C1∼C6 ester is specifically 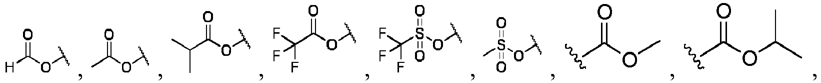 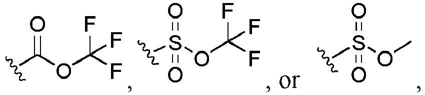 etc.

In some specific embodiments of the present invention, the above -NR₁₁R₁₁ₐ is specifically amino, 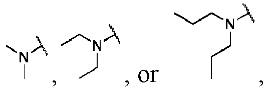 etc.

In some specific embodiments of the present invention, the above 3-14 membered heterocyclyl containing 1-4 heteroatoms selected from the group consisting of N, O, S, SO and SO₂ is specifically 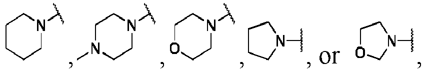 etc.

Preferably in the present invention, R₁₀ is selected from the group consisting of hydrogen, hydroxyl, C1∼C6 alkyl, C1∼C6 alkoxy and C1∼C6 alkylthio.

Further preferably, R₁₀ is selected from the group consisting of hydrogen, hydroxyl, C1-C3 alkyl, C1-C3 alkoxy and C1~C3 alkylthio.

The C1-C3 alkyl, C1-C3 alkoxy or C1-C3 alkylthio may be further substituted by one or more substituents selected from the group consisting of halogen, hydroxyl, amino, carboxyl, cyano and nitro.

Further preferably, R₁₀ is selected from the group consisting of hydrogen, hydroxyl, methyl, ethyl, n-propyl, isopropyl, n-butyl, isobutyl, tert-butyl, n-pentyl, isopentyl, n-hexyl, isohexyl, hydroxymethyl, hydroxyethyl, methoxy, ethoxy, n-propyloxy, isopropyloxy, n-butyloxy, isobutyloxy, tert-butyloxy, n-pentyloxy, isopentyloxy, n-hexyloxy, methylthio, ethylthio, n-propylthio, isopropylthio, n-butylthio, isobutylthio, tert-butylthio, n-pentylthio, isopentylthio and n-hexylthio.

In some specific embodiments of the present invention, the glycoside derivative is a compound having any one of the following structures or a pharmaceutically acceptable salt thereof:

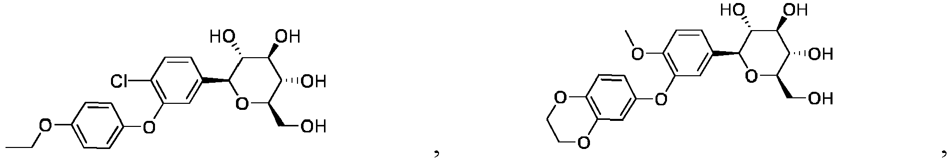

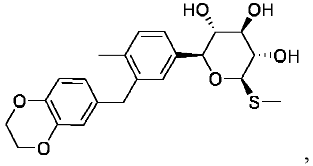

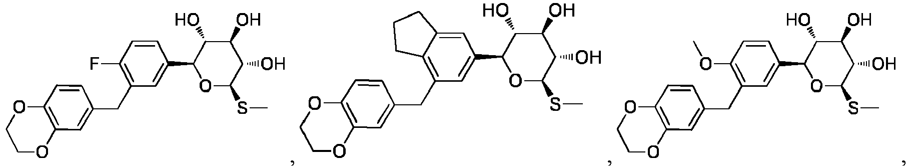

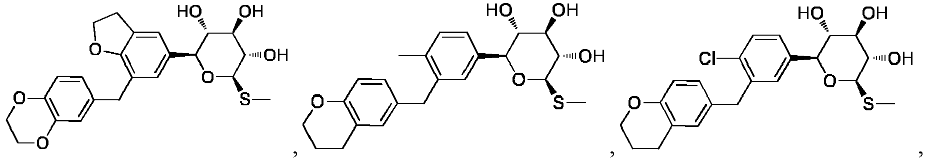

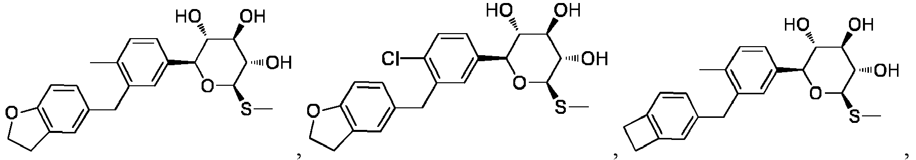

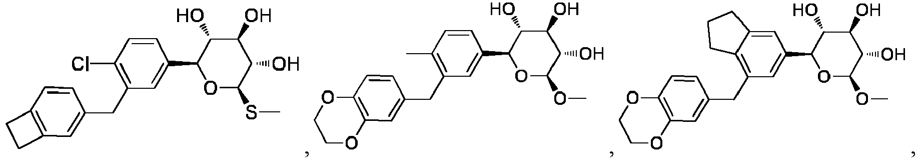

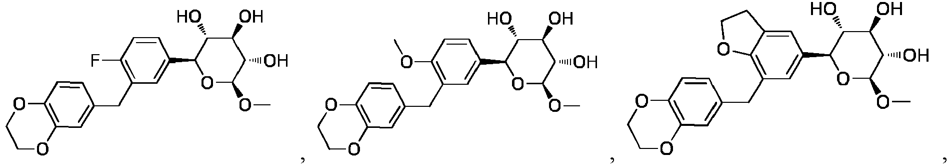

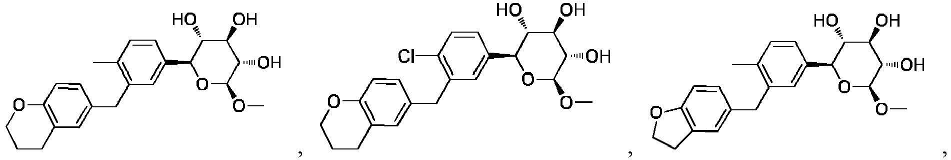

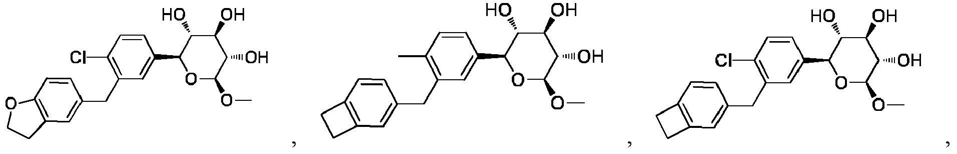

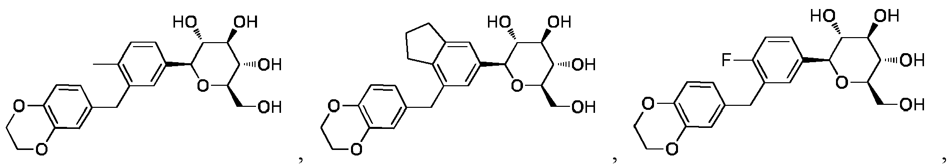

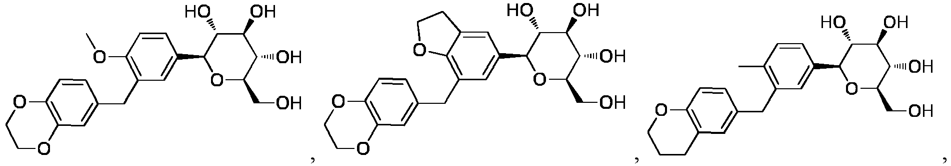

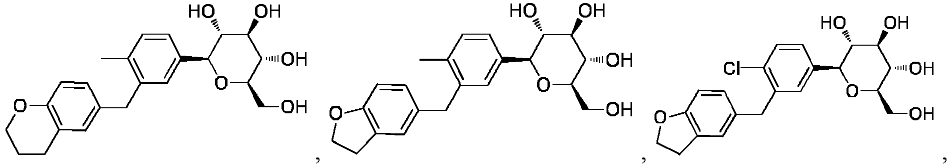

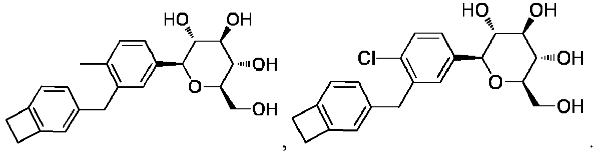

In some specific embodiments of the present invention, the glycoside derivative is a compound having any one of the following structures or a pharmaceutically acceptable salt thereof:

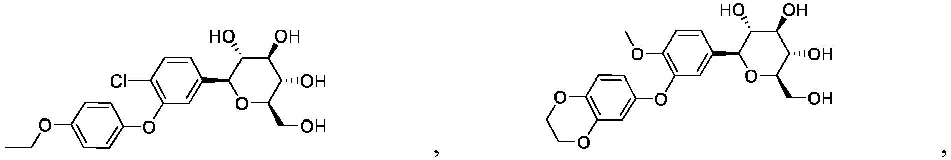

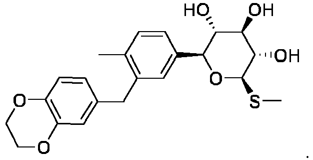

In some specific embodiments of the present invention, the glycoside derivative is a compound having the following structure or a pharmaceutically acceptable salt thereof:

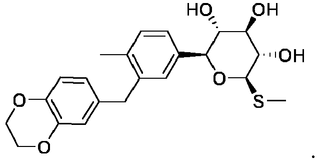

The second object of the present invention is to provide a method for preparing the above glycoside derivative, comprising:
subjecting a compound represented by formula III to a deprotection reaction to prepare a compound represented by formula I-a; 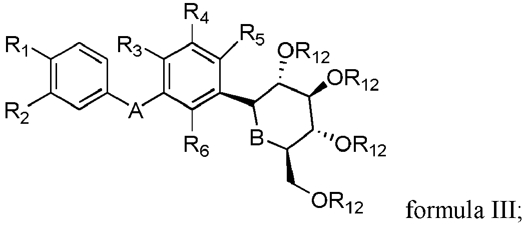 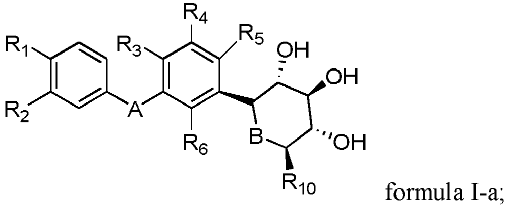
wherein, A, B, R₁, R₂, R₃, R₄, R₅ and R₆ are defined as the same as above, and will not be repeated here.
R₁₀ is hydroxymethyl.
R₁₂ is alkyl (exemplarily, alkyl is C1~C6 alkyl, for example, methyl, ethyl, n-propyl, isopropyl, n-butyl, isobutyl, tert-butyl, n-pentyl, isopentyl, n-hexyl, isohexyl, etc.), TMS-(trimethylsilyl), Bn-(benzyl), formyl, Ac-(acetyl), THP-(tetrahydropyranyl), MOM-(methoxymethyl) or TBDMS-(tert-butyldimethylsilyl).

Exemplarily, in the case that R₁₂ is TMS- or TBDMS-, a reagent for the deprotection is TBAF; in the case that R₁₂ is Bn-, the deprotection reaction is carried out under a condition of H₂/Pd-C, H₂/Pt-C, or H₂/Pd(OH)₂-C, etc.; in the case that R₁₂ is Ac-, the deprotection reaction is carried out under a strong base condition (such as an aqueous solution of sodium hydroxide, or an aqueous solution of potassium hydroxide) or a strong acid condition; in the case that R₁₂ is THP- or MOM-, the deprotection reaction is carried out under an acidic condition (such as an aqueous solution of hydrochloric acid, an ethyl acetate solution of hydrogen chloride (HCl(g)/EtOAc), a methanol solution of hydrogen chloride (HCl(g)/CH₃OH), an ethanol solution of hydrogen chloride (HCl(g)/EtOH), or a dioxane solution of hydrogen chloride (HCl(g)/dioxane)); in the case that R₁₂ is methyl and the like, a reagent for the deprotection is selected from the group consisting of concentrated hydrochloric acid, hydrobromic acid, concentrated sulfuric acid and boron tribromide.

In the above preparation method, as a preferred embodiment, the compound represented by formula III is prepared from a compound represented by formula IV; 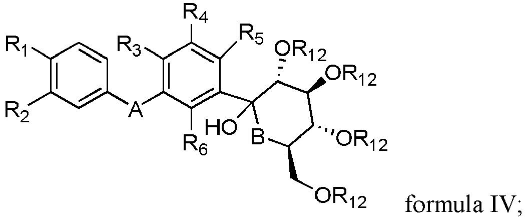 wherein, A, B, R₁, R₂, R₃, R₄, R₅, R₆ and R₁₂ are defined as the same as above, and will not be repeated here.

Exemplarily, the reaction conditions for preparing the compound represented by formula III from the compound represented by formula IV include BF₃.Et₂O.

In the above preparation method, as a preferred embodiment, the compound represented by formula IV is prepared by reacting a compound represented by formula V with a compound represented by formula VI; 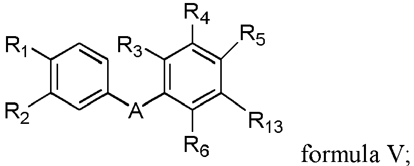 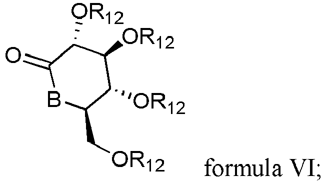
wherein, A, B, R₁, R₂, R₃, R₄, R₅, R₆ and R₁₂ are defined as the same as above, and will not be repeated here.
R₁₃ is selected from the group consisting of -H, -F, -Cl, -Br, -I, -OMs, -OTs and -OTf.

Exemplarily, a reaction reagent used for preparing the compound represented by formula IV from the compound represented by formula V and the compound represented by formula VI is LDA (lithium diisopropylamide), or n-BuLi (n-butyllithium), etc.

In some specific embodiments of the present invention, the above glycoside derivative is prepared by the following reaction route:

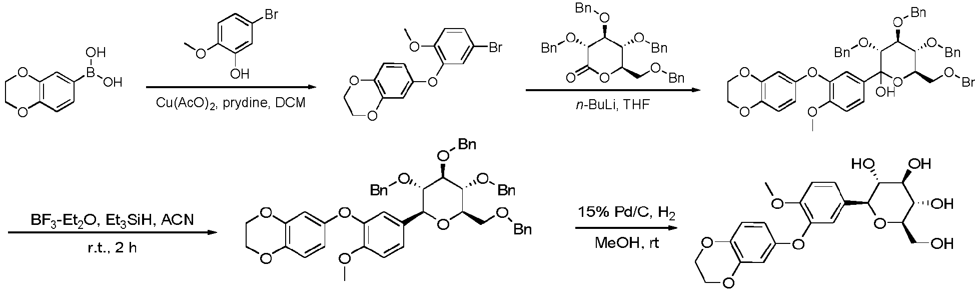

In some specific embodiments of the present invention, the above glycoside derivative is prepared by the following reaction route:

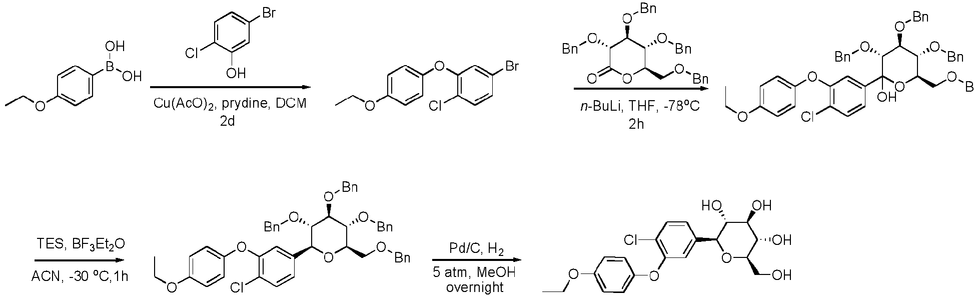

The present invention provides another method for preparing the above glycoside derivative, comprising:
subjecting a compound represented by formula VII to sulfurization reaction and deprotection reaction to prepare a compound represented by formula I-a; 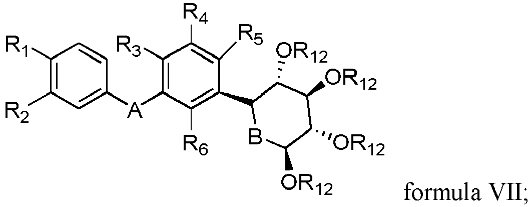 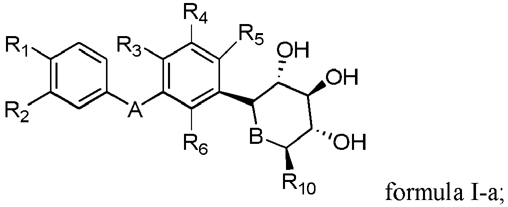
wherein, A, B, R₁, R₂, R₃, R₄, R₅ and R₆ are defined as the same as above, and will not be repeated here.
R₁₀ is methylthio.
Preferably, B is an O atom.
R₁₂ is alkyl (exemplarily, alkyl is C1~C6 alkyl, for example, methyl, ethyl, n-propyl, isopropyl, n-butyl, isobutyl, tert-butyl, n-pentyl, isopentyl, n-hexyl, isohexyl, etc.), TMS-(trimethylsilyl), Bn-(benzyl), formyl, Ac-(acetyl), THP-(tetrahydropyranyl), MOM-(methoxymethyl) or TBDMS-(tert-butyldimethylsilyl).

Exemplarily, a sulfurization reagent for the sulfurization reaction is thiourea.

Exemplarily, in the case that R₁₂ is TMS- or TBDMS-, a reagent for the deprotection is TBAF; in the case that R₁₂ is Bn-, the deprotection reaction is carried out under a condition of H₂/Pd-C, H₂/Pt-C, or H₂/Pd(OH)₂-C, etc.; in the case that R₁₂ is Ac-, the deprotection reaction is carried out under a strong base condition (such as an aqueous solution of sodium hydroxide, or an aqueous solution of potassium hydroxide) or a strong acid condition; in the case that R₁₂ is THP- or MOM-, the deprotection reaction is carried out under an acidic condition (such as an aqueous solution of hydrochloric acid, an ethyl acetate solution of hydrogen chloride (HCl(g)/EtOAc), a methanol solution of hydrogen chloride (HCl(g)/CH₃OH), an ethanol solution of hydrogen chloride (HCl(g)/EtOH), or a dioxane solution of hydrogen chloride (HCl(g)/dioxane)); in the case that R₁₂ is methyl and the like, a reagent for the deprotection is selected from the group consisting of concentrated hydrochloric acid, hydrobromic acid, concentrated sulfuric acid and boron tribromide.

In the above preparation method, as a preferred embodiment, the compound represented by formula VII is prepared from a compound represented by formula VIII; 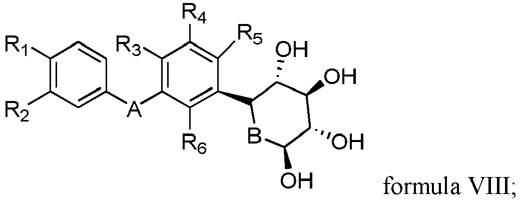 wherein, A, B, R₁, R₂, R₃, R₄, R₅ and R₆ are defined as the same as above, and will not be repeated here.

Exemplarily, the reaction conditions for preparing the compound represented by formula VII from the compound represented by formula VIII include AC₂O, DMAP and TEA.

In the above preparation method, as a preferred embodiment, the compound represented by formula VIII is prepared from a compound represented by formula IX; 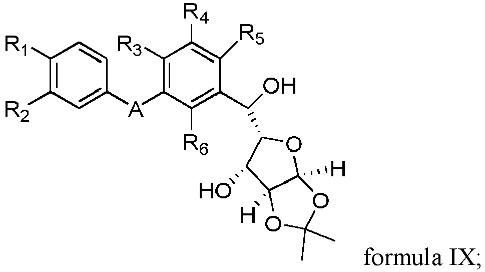 wherein, A, R₁, R₂, R₃, R₄, R₅ and R₆ are defined as the same as above, and will not be repeated here.

Exemplarily, the conditions for preparing the compound represented by formula VIII from the compound represented by formula IX include AcOH and H₂O.

In the above preparation method, as a preferred embodiment, the compound represented by formula IX is prepared by reacting the compound represented by formula V with a compound represented by formula X followed by a reduction reaction; 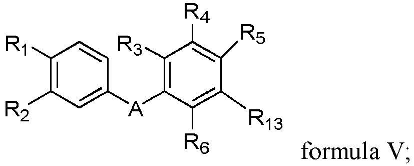 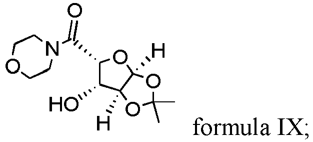 wherein, A, R₁, R₂, R₃, R₄, R₅ and R₆ are defined as the same as above, and will not be repeated here.

R₁₃ is selected from the group consisting of -H, -F, -Cl, -Br, -I, -OMs, -OTs and -OTf.

Exemplarily, a catalyst used for the reaction of the compound represented by formula V with the compound represented by formula X is t-BuMgCl.

Exemplarily, the conditions for the above reduction reaction include CeCl₃ and NaBH₄.

In some specific embodiments of the present invention, the above glycoside derivative is prepared by the following reaction route:

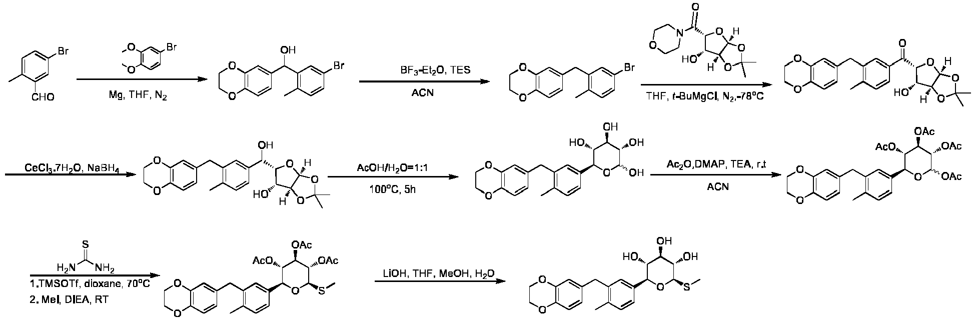

The third object of the present invention is to provide an intermediate compound represented by formula IV or a salt thereof:

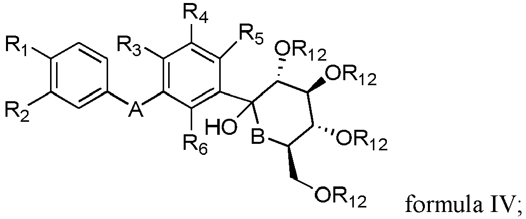

wherein, A, B, R₁, R₂, R₃, R₄, R₅, R₆ and R₁₂ are defined as the same as above, and will not be repeated here.

The present invention further provides another intermediate compound represented by formula IX or a salt thereof: 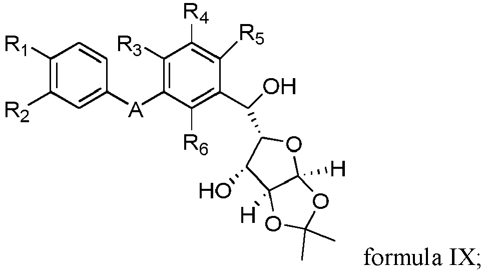 wherein, A, R₁, R₂, R₃, R₄, R₅ and R₆ are defined as the same as above, and will not be repeated here.

In some specific embodiments of the present invention, the above intermediate has any one of the following structures:

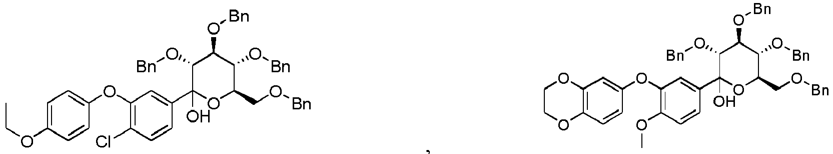

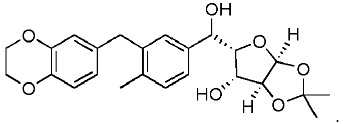

The fourth object of the present invention is to provide a pharmaceutical composition, comprising the above glycoside derivative or the glycoside derivative prepared by the above method, and a pharmaceutically acceptable carrier, excipient, diluent, adjuvant, vehicle or a combination thereof.

The present invention has no special limitation on types of the carrier, excipient, diluent, adjuvant, and vehicle, which may be applicable carriers, excipients, diluents, adjuvants, and vehicles well known to those skilled in the art.

The fifth object of the present invention is to provide use of the above glycoside derivative, the glycoside derivative prepared by the above method, or the above pharmaceutical composition in the manufacture of a medicament for preventing, treating or alleviating diabetes mellitus and a complication thereof.

In the present invention, the above diabetes mellitus may be selected from the group consisting of type I diabetes mellitus and type II diabetes mellitus, preferably type II diabetes mellitus.

The above complication is preferably a cardiovascular disease caused by type II diabetes mellitus.

The above glycoside derivative, the glycoside derivative prepared by the above method, or the above pharmaceutical composition provided by the present invention may be used to improve blood glucose control or protect cardiovascular system in adult patients with type 2 diabetes mellitus without causing the risk of hypoglycemia, thereby achieving rapid and safe hypoglycemic effect.

In the present invention, the above glycoside derivative may be used in combination with other drugs for preventing, treating or alleviating diabetes mellitus and a complication thereof.

The sixth object of the present invention is to provide a method for preventing, treating or alleviating diabetes mellitus and a complication thereof, comprising contacting the above glycoside derivative, the glycoside derivative prepared by the above method, or the above pharmaceutical composition with a biological specimen or a patient in need thereof. In a specific aspect, the method for preventing, treating or alleviating diabetes mellitus and a complication thereof comprises administering the glycoside derivative, the glycoside derivative prepared by the above method, or the above pharmaceutical composition of the present invention to a patient in need thereof.

Compared with the prior art, the present invention provides a glycoside derivative represented by formula I or a pharmaceutically acceptable salt thereof.

Compared with the prior art, the present invention has the following technical effects:
1. The present invention provides a novel glycoside derivative, which has a therapeutic effect on type 2 diabetes mellitus. Compared with the prior art, the glycoside derivative of the present invention has excellent therapeutic effects on type II diabetes mellitus at high, medium and low doses.
2. The method for preparing the glycoside derivative of the present invention adopts cheap and easily available chemical products as starting materials, and has high synthesis yield in each step. Therefore, the method has low production cost and is more suitable for industrial production.

### DETAILED DESCRIPTION

### Terms:

Unless otherwise stated, the terms used in the present invention, including the terms used in the specification and claims, are defined as follows. It must be noted that in the specification and appended claims, nouns not modified by a numeral generally include the plural unless the context clearly indicates otherwise. Unless otherwise stated, conventional methods of mass spectrometry, nuclear magnetic resonance, HPLC, biochemistry and pharmacology are used.

The term "alkyl" used herein is intended to include both branched and straight-chain saturated aliphatic hydrocarbon groups having the indicated number of carbon atoms. For example, "C1-C6 alkyl" refers alkyl having 1 to 6 carbon atoms. Examples of alkyl include, but are not limited to, methyl (Me), ethyl (Et), propyl (such as n-propyl and isopropyl), butyl (such as n-butyl, isobutyl and tert-butyl), and pentyl (such as n-pentyl, isopentyl and neopentyl). Preferred Alkyl is preferably C1-C6 alkyl, more preferably C1-C4 alkyl.

The term "alkoxy" or "alkyloxy" refers to -O-alkyl. "C1-C6 alkoxy" (or alkyloxy) is intended to include C1, C2, C3, C4, C5 and C6 alkoxy. Alkoxy is preferably C1-C6 alkoxy, more preferably C1-C4 alkoxy. Examples of alkoxy include, but are not limited to, methoxy, ethoxy, propoxy (such as n-propoxy and isopropoxy), and tert-butoxy. Similarly, "alkylthio" or "thioalkoxy" refers to alkyl as defined above having the indicated number of carbon atoms and connected through a sulfur bridge; for example, methyl-S- and ethyl-S-.

The term "carbonyl" refers to an organic functional group consisting of two atoms, carbon and oxygen, linked by a double bond (C=O).

The term "aryl" refers to a monocyclic, bicyclic or tricyclic ring system having a total of 5 to 12 ring members, wherein at least one ring in the system is aromatic and each ring contains 3 to 7 ring members. A monocyclic aryl refers to phenyl, and a bicyclic aryl or an aryl with more rings refers to naphthyl, anthracenyl, etc., where the bicyclic aryl may also be a benzene ring fused with cycloalkyl, cycloalkenyl, or cycloalkynyl. Preferred aryl is C6-C12 aryl. In certain embodiments of the present invention, "aryl" refers to an aromatic ring system including but not limited to phenyl, biphenyl, indanyl, 1-naphthyl, 2-naphthyl, and tetrahydronaphthyl. The term "aralkyl" or "arylalkyl" refers to an alkyl residue attached to an aromatic ring. Non-limiting examples include benzyl, phenethyl, and the like. Fused aryl may be attached to another group at a suitable position on the cycloalkyl ring or aromatic ring. For example, an arrowed line drawn from a ring system indicates a bond that may be attached to any suitable ring atom.

The term "cycloalkyl" refers to a monocyclic or bicyclic alkyl. Monocyclic alkyl refers to C3-C8 cyclic alkyl, including but not limited to cyclopropyl, cyclobutyl, cyclopentyl, cyclohexyl and norbornyl. Branched cycloalkyl such as 1-methylcyclopropyl and 2-methylcyclopropyl are included within the definition of "cycloalkyl". Bicyclic alkyl includes bridged-, spiro-, or fused- cycloalkyl. Preferred cycloalkyl is C3-C6 cycloalkyl.

"Halo" or "halogen" includes fluorine, chlorine, bromine and iodine. "Haloalkyl" is intended to include branched and straight-chain saturated aliphatic hydrocarbon groups having the indicated number of carbon atoms and substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl also include "fluoroalkyl" intended to include branched and straight-chain saturated aliphatic hydrocarbon groups having the indicated number of carbon atoms and substituted with one or more fluorine atoms.

"Haloalkoxy" or "haloalkyloxy" refers to haloalkyl as defined above having the indicated number of carbon atoms and connected through an oxygen bridge. For example, "C1-C6 haloalkoxy" is intended to include C1, C2, C3, C4, C5 and C6 haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" refers to haloalkyl as defined above having the indicated number of carbon atoms and attached through a sulfur bridge, for example trifluoromethyl-S- and pentafluoroethyl-S-.

The term "heteroaryl" refers to a stable 3-, 4-, 5-, 6-, or 7-membered aromatic monocycle or aromatic bicycle, or a 7-, 8-, 9-, 10-, 11-, 12-, 13- or 14-membered aromatic heteropolycycle, which is fully unsaturated or partially unsaturated, contains carbon atoms and 1, 2, 3 or 4 heteroatoms independently selected from the group consisting of N, O and S, and includes any one of the following polycyclic groups, where any heterocycle defined above is fused to a benzene ring. Nitrogen and sulfur atoms may be optionally oxidized. Nitrogen atom is substituted or unsubstituted (i.e. N or NR, where R is H or another substituent if defined). A heterocycle may be attached to its side group at any heteroatom or carbon atom that results in a stable structure. If the resulting compound is stable, the heterocyclyl described herein may be substituted on carbon or nitrogen atoms. The nitrogen in the heterocycle may optionally be quaternized. Preferably, in the case that the total number of S and O atoms in the heterocycle exceeds 1, these heteroatoms are not adjacent to each other. Preferably, the total number of S and O atoms in the heterocycle is not greater than 1. Preferred heteroaryl is 5-12 membered heteroaryl. Examples of heteroaryl include, but are not limited to, acridinyl, azetidinyl, azocinyl, benzimidazolyl, benzofuryl, benzothiofuryl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofuro[2,3-b]tetrahydrofuryl, furyl, furazanyl, imidazolidinyl, imidazolinyl, imidazolyl, 1H-indazolyl, imidazopyridyl, indolenyl, dihydroindolyl, indolizinyl, indolyl, 3H-indolyl, isatinoyl, isobenzofuryl, isochromanyl, isoindazolyl, isodihydroindolyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridyl, oxazolidinyl, perimidinyl, hydroxyindolyl, pyrimidinyl, phenanthridinyl, phenanthrolinyl, phenazinyl, phenothiazinyl, phenoxathiyl, phenoxazinyl, phthalazinyl, piperazinyl, piperidinyl, piperidinonyl, 4-piperidinonyl, piperonyl, pteridinyl, purinyl, pyranyl, pyrazinyl, pyrazolidinyl, pyrazolinyl, pyrazolopyridyl, pyrazolyl, pyridazinyl, pyridoxazolyl, pyridimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolinyl, 4H-quinazinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthryl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl, xanthenyl, quinolinyl, isoquinolyl, phthalazinyl, quinazolinyl, indolyl, isoindolyl, dihydroindolyl, 1H-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuryl, chromanyl, 1,2,3,4-tetrahydroquinoxalinyl and 1,2,3,4-tetrahydroquinazolinyl. Preferred heteroaryl in the present invention is C2-C12 heteroaryl or C5-C12 heteroaryl.

The term "heterocyclyl" or "heterocycloalkyl" used herein refers to a monocyclic heterocycloalkyl system, or a bicyclic heterocycloalkyl system. Monocyclic heterocycloalkyl refers to a 3-8 membered saturated or unsaturated non-aromatic cyclic alkyl system containing at least one heteroatom selected from the group consisting of O, N, S, and P. Preferred heterocyclyl is 3-12 membered heterocyclyl or 3-6 membered heterocyclyl; and preferred heterocyclyl is -O-C2~C6 heterocyclyl.

The term "substituted" used herein means that at least one hydrogen atom is replaced by a non-hydrogen group, provided that normal valences are maintained and that the substitution results in a stable compound.

When any variable occurs more than once in any composition or formula of a compound, its definition at each occurrence is independent of its definition at every other occurrence. Thus, for example, if a group is shown to be substituted with 0, 1, 2 or 3 R groups, the group may optionally be substituted with up to three R groups, and R at each occurrence is independently selected from the definition of R. Moreover, combinations of substituents and/or variables are permissible only if such combinations result in stable compounds.

The term "patient" used herein refers to an organism being treated by the methods of the present invention. Such organisms preferably include, but are not limited to, mammals (such as such as rodents, apes/monkeys, horses, cattle, pigs, dogs, cats, etc.), and most preferably humans.

The term "effective amount" used herein refers to an amount of a drug or agent (i.e., the compound of the present invention) that will elicit a biological or medical response in a tissue, system, animal or human sought by, for example, a researcher or clinician. Furthermore, the term "therapeutically effective amount" refers to an amount which results in improved treatment, cure, prevention, or alleviation of a disease, disorder or side effect, or reduction in the rate of disease or disorder progression compared to the corresponding subjects who are not received the above amounts. An effective amount may be given in one or more administrations, applications or doses and is not intended to be limited by a particular formulation or route of administration. The term further includes an effective amount for enhancing normal physiological function within its scope.

The term "treatment" used herein includes any effects that result in improvement of a condition, disease, disorder and the like, such as alleviation, reduction, regulation, mitigation, elimination, or amelioration of the symptoms. As used herein, the improvement of a certain disease, symptom or condition caused by administering a certain compound or pharmaceutical composition particularly refers to improving the severity, delaying the onset, slowing down the progression of the disease, or shortening the duration of the disease, which may be attributed to administration or the circumstances related to administration whether the administration is regular or temporary, or continuous or intermittent. As used herein, the term "prevention" refers to preventing, blocking, eliminating the occurrence of a disease or interfering with or slowing down the progression of a disease before the occurrence of the disease or symptom.

The term "pharmaceutical composition" used herein refers to a combination of an active agent with an inert or active carrier, making the composition especially suitable for use in *in vivo* or *ex vivo* diagnosis or therapy.

The term "pharmaceutical" or "pharmaceutically acceptable" are used herein to refer to such compounds, substances, compositions and/or dosage forms that, within the scope of sound medical evaluation, are suitable for use in contacting with the tissues of humans and animals without undue toxicity, irritation, allergic reaction and/or other problems or complications, and commensurate with a reasonable benefit/risk ratio.

The phrase "pharmaceutically acceptable carrier" or "pharmaceutical carrier" used herein refers to a pharmaceutical substance, composition or vehicle, such as a liquid or solid filler, diluent, excipient, manufacturing auxiliary (e.g. a lubricant, talc, magnesium stearate, calcium stearate, zinc stearate or stearic acid) or a solvent-encapsulating substance, which is involved in carrying or transporting an active compound from one organ or body part to another organ or body part. Each carrier must be "acceptable" in terms of being compatible with the other ingredients of the formulation and harmless to the patient.

In order to further illustrate the present invention, the glycoside derivative and preparation method thereof and uses thereof provided by the present invention are described in detail below in conjunction with examples.

### Preparation Examples:

### Example 1 Preparation of Compound 1

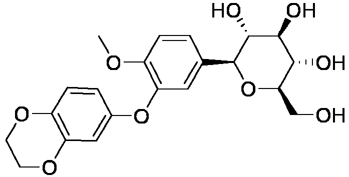

(1) Under stirring, a solution of 2,3-dihydro-1,4-benzodioxin-6-ylboronic acid (2.30 g, 12.806 mmol, 1.3 eq) and 5-bromo-2-methoxyphenol (2.00 g, 9.851 mmol, 1.00 eq) in dichloromethane (50.00 mL) were added with Cu(AcO)₂ (1.97 g, 10.836 mmol, 1.1 eq) and pyridine (2.34 g, 29.552 mmol, 3 eq). The resulting mixture was stirred under an air atmosphere for 48 h at room temperature. 100 mL of H₂O was added to the above mixture. The resulting mixture was extracted with DCM (3×100 mL). The combined organic phase was washed with saturated brine (1×100 mL) and dried over anhydrous sodium sulfate. After filtration, the filtrate was concentrated under reduced pressure. The residue was purified by silica gel column chromatography and eluted with PE/EA (5:1) to obtain a grey-white solid of 6-(5-bromo-2-methoxyphenoxy)-2,3-dihydro-1,4-benzodioxane (1134 mg with a yield of 34.14%).

The reaction route is as follows:

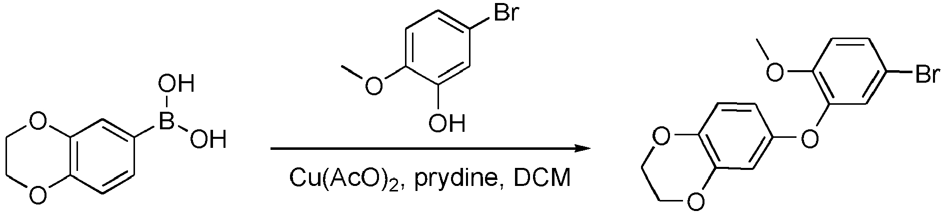

(2) Under nitrogen protection and stirring, n-BuLi (0.24 mL, 0.594 mmol, 2.00 eq) was dropwise added to a solution of 6-(5-bromo-2-methoxyphenoxy)-2,3-dihydro-1,4-benzodioxane (100.00 mg, 0.297 mmol, 1.00 eq) in tetrahydrofuran (1.00 mL) at -78°C. The resulting mixture was stirred at -78°C under a nitrogen atmosphere for 1 h. The above mixture was dropwise added with a solution of (3R,4S,5R,6R)-3,4,5-tris(benzyloxy)-6-[(benzyloxy)methyl]oxan-2-one (479.26 mg, 0.890 mmol, 3.00 eq) in tetrahydrofuran (1.00 mL) at -78°C. The resulting mixture was stirred at -78°C for another 1 h. After the system was heated to room temperature, the reaction was quenched with saturated NH₄Cl (aq.) at room temperature, and the resulting mixture was extracted with EA (2×3 mL). The combined organic phase was dried over anhydrous sodium sulfate. After filtration, the filtrate was concentrated by distillation under reduced pressure. The crude product was used directly in the next step without further purification.

The reaction route is as follows:

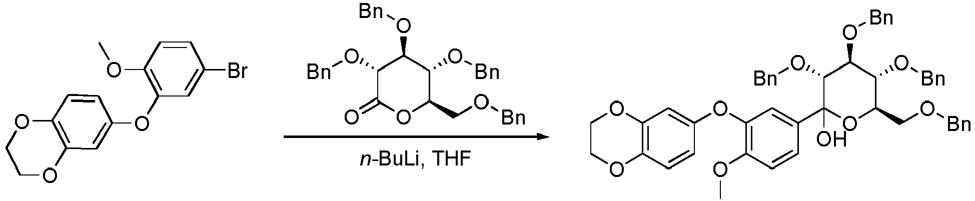

(3) Under nitrogen protection and stirring, BF₃-Et₂O was dropwise added to a solution of (3R,4S,5R,6R)-3,4,5-tris(benzyloxy)-6-[(benzyloxy)methyl]-2-[3-(2,3-dihydro-1,4-benzodiox in-6-oxyl)-4-methoxyphenyl]oxan-2-ol (540.00 mg, 1.00 eq) and TES (157 mg) in acetonitrile (4 ml) at -30°C for 30 min of reaction. The reaction was monitored by LCMS. After the reaction was completed, the reaction was quenched with K₂CO₃ (aq) for 1 h at room temperature. The resulting mixture was extracted with EtOAc (3x20 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of acetonitrile-water, a gradient of 40%-85%, an elution time of 10 min, and a detector of UV 254 nm. A grey-white solid of 6-[2-methoxy-5-[(2R,3S,4R,5R,6R)-3,4,5-tris(benzyloxy)-6-[(benzyloxy)methyl]oxane-2-yl]p henoxy]-2,3-dihydro-1,4-benzodioxane (470 mg with a yield of 85.9%) was obtained.

The reaction route is as follows:

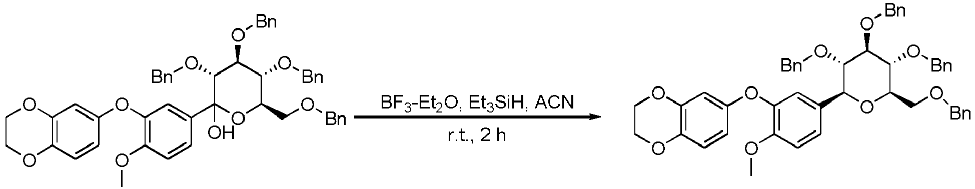

(4) A solution of 6-[2-methoxy-5-[(2R,3S,4R,5R,6R)-3,4,5-tris(benzyloxy) -6-[(benzyloxy)methyl]oxy-2-yl]phenoxy]-2,3-dihydro-1,4-benzodioxane (500.00 mg, 1.00 eq) and 15% Pd/C in methanol was stirred at 50°C under a hydrogen atmosphere overnight. The resulting mixture was filtered, and the filtrate was concentrated under reduced pressure. The residue was purified by reverse phase flash evaporation chromatography under the following conditions: a column of C18 silica gel column, a mobile phase of acetonitrile-water, a gradient of 10%-50%, an elution time of 30 min, and a detector of UV 254 nm. A grey-white solid of (2S,3R,4R,5S,6R)-2-[3-(2,3-dihydro-1,4-benzodioxin-6-oxyl)-4-methoxyphenyl]-6-(hydroxy methyl)oxane-3,4,5-triol (140 mg with a yield of 52.0%) was obtained.

The reaction route is as follows: 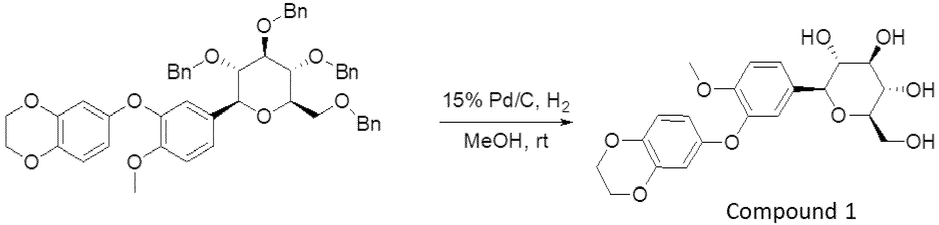 LC-MS: (ES, m/z): [M-1] =419;
¹H NMR: (400 MHz, Methanol-*d*₄) δ 7.22 (dd, *J=* 8.4, 2.1 Hz, 1H), 7.13 - 7.03 (m, 2H), 6.74 (d, *J* = 8.6 Hz, 1H), 6.44 - 6.36 (m, 2H), 4.24 - 4.16 (m, 4H), 4.07 (d, *J* = 9.4 Hz, 1H), 3.88 (dd, *J* = 12.0, 1.5 Hz, 1H), 3.80 (s, 3H), 3.76 - 3.65 (m, 1H), 3.51 - 3.32 (m, 5H).

### Example 2 Preparation of Compound 2

(1) A solution of 6-bromo-2,3-dihydro-1,4-benzodioxane (4.40 g, 1.00 eq) and Mg (2.00 g, 5.00 eq) in THF (15 ml) was stirred at 50°C under a nitrogen atmosphere. The resulting mixture was stirred at room temperature under a nitrogen atmosphere for 2 h. The above mixture was dropwise added with 5-bromo-2-methylbenzaldehyde (3.00 g, 1.00 eq) within 10 min while the system was maintained at 0°C. The resulting mixture was further stirred at room temperature for 2 h. The reaction was monitored by LCMS. After the reaction was completed, the reaction was quenched at 0°C with an ice-water mixture. The resulting mixture was extracted with EtOAc (3x30 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of acetonitrile-water, a gradient of 20%-70%, an elution time of 15 min, and a detector of UV 254 nm. A grey-white solid of (5-bromo-2-methylphenyl)(2,3-dihydro-1,4-benzodioxin-6-yl)methanol (4.8 g with a yield of 69.6%) was obtained.

The reaction route is as follows:

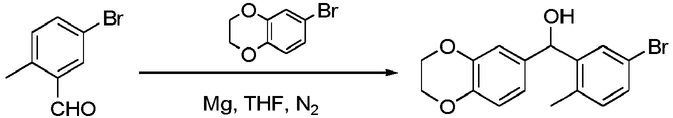

(2) (5-Bromo-2-methylphenyl)(2,3-dihydro-1,4-benzodioxin-6-yl)methanol (4.80 g, 1.00 eq) and TES (3.4 g, 2.0 eq) were added into acetonitrile (80 mL) to form a mixture, and the mixture was then dropwise added with BF₃-Et₂O at -30°C under a nitrogen atmosphere and stirring. The resulting mixture was stirred at -30°C for 1 h under a nitrogen atmosphere. The reaction was monitored by LCMS. After the reaction was completed, the reaction was quenched with K₂CO₃ at -20°C. The resulting mixture was extracted with EtOAc (3x70 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of MeOH-water, a gradient of 20%-60%, an elution time of 25 min, and a detector of UV 254 nm. A grey-white solid of 6-[(5-bromo-2-methylphenyl)methyl]-2,3-dihydro-1,4-benzodioxane (3.6 g with a yield of 78.0%) was obtained.

The reaction route is as follows:

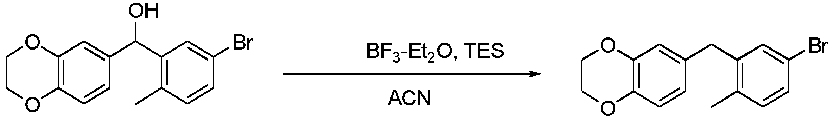

(3) Under stirring and nitrogen protection, a mixture of 6-[(5-bromo-2-methylphenyl)methyl]-2,3-dihydro-1,4-benzodioxane (3.60 g, 1.20 eq) in tetrahydrofuran was dropwise added with n-BuLi (1.2 eq) at -78°C.

Under stirring and nitrogen protection, a mixture of (3aS,5S,6S,6aS)-2,2-dimethyl-5-(morpholine-4-carbonyl)-tetrahydrofuran[2,3-d][1,3]dioxol-6 -ol (2.50 g, 1.00 eq) in tetrahydrofuran (15 mL) was dropwise added with t-BuMgCl (6.1 mL) at 0°C within 30 min.

The solutions obtained above were mixed and stirred at -78°C for 30 min of reaction. The reaction was monitored by LCMS. After the reaction was completed, NH₄Cl (aq.) (5 mL) was added at room temperature to quench the reaction. The resulting mixture was extracted with EtOAc (3x50 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of acetonitrile-water, a gradient of 15%-60%, an elution time of 20 min, and a detector of UV 254 nm. A grey-white solid of (3aR,5S,6R,6aR)-5-[3-(2,3-dihydro-1,4-benzodioxin-6-ylmethyl)-4-methylbenzoyl]-2,2-dimet hyl-tetrahydrofuran[2,3-d][1,3]dioxin-6-ol (4.6 g with a yield of 95.0%) was obtained.

The reaction route is as follows:

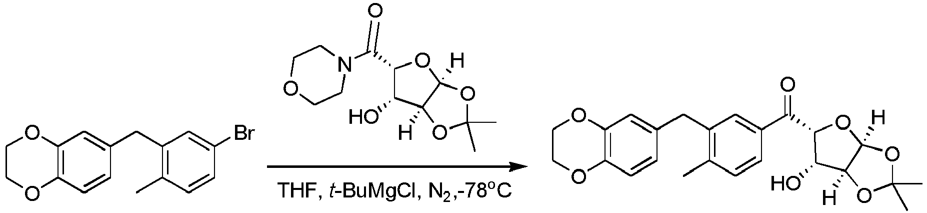

(4) Under nitrogen protection and stirring, a solution of (3aR,5S,6R,6aR)-5-[3-(2,3-dihydro-1,4-benzodioxin-6-ylmethyl)-4-methylbenzoyl]-2,2-dimet hyl-tetrahydrofuran[2,3-d][1,3]dioxol-6-ol (4.60 g, 1.00 eq) and CeCl₃·7H₂O (5.20 g, 1.30 eq) in methanol (20 mL) was added with NaBH₄ (0.50 g, 1.20 eq) for 30 min of reaction at -30°C. The reaction was monitored by LCMS. After the reaction was completed, HCl (2 N) was added at -30°C to quench the reaction. The resulting mixture was extracted with EtOAc (3×40 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of acetonitrile-water, a gradient of 10%-50%, an elution time of 10 min, and a detector of UV 254 nm. A grey-white solid of (3aR,5R,6S,6aR)-5-[[3-(2,3-dihydro-1,4-benzodioxin-6-ylmethyl)-4-methylphenyl](hydroxy) methyl]-2,2-dimethyl-tetrahydrofuran[2,3-d][1,3]dioxin-6-ol (2.6 g with a yield of 56.2%) was obtained.

The reaction route is as follows:

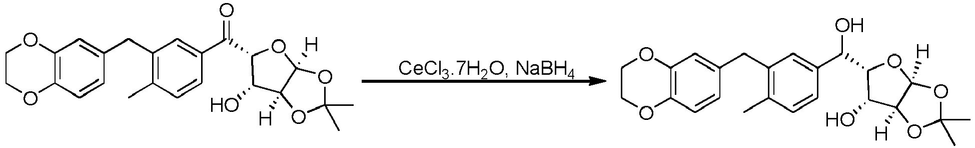

(5) A solution of (3aR,5R,6S,6aR)-5-[[3-(2,3-dihydro-1,4-benzodioxin-6-ylmethyl) -4-methylphenyl](hydroxyl)methyl]-2,2-dimethyl-tetrahydrofuran[2,3-d][1,3]dioxol-6-ol (2.60 g, 1.00 eq) and AcOH (20 mL) in H₂O (20 mL) was stirred at 100°C for 4 h of reaction. The reaction was monitored by LCMS. After the reaction was completed, the reaction system was concentrated under reduced pressure, and the obtained concentrate was extracted with EtOAc (3×100 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of acetonitrile-water, a gradient of 10%-40%, an elution time of 15 min, and a detector of UV 254 nm. A grey-white solid of (2S,3S,4R,5R)-6-[3-(2,3-dihydro-1,4-benzodioxin-6-ylmethyl)-4-methylphenyl]oxane-2,3,4,5 -tetraol (2.25 g with a yield of 95.0%) was obtained.

The reaction route is as follows:

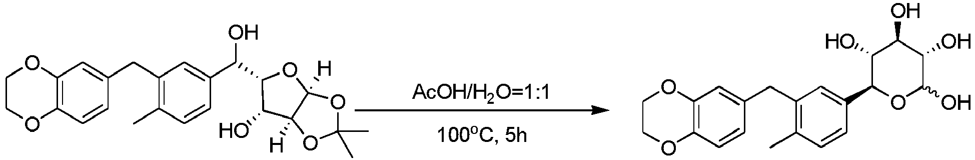

(6) Under stirring, a solution of (2S,3S,4R,5R)-6-[3-(2,3-dihydro-1,4-benzodioxin-6-ylmethyl)-4-methylphenyl]oxane-2,3,4,5 -tetraol (2.25 g, 1.00 eq) and Ac₂O (3.55 g) in acetonitrile (20 mL) was added with TEA (3.52 g, 6.00 eq) and DMAP (8.00 mg, 0.01 eq), and stirred at room temperature for 1.5 h of reaction. The reaction was monitored by LCMS. After the reaction was completed, the resulting mixture was extracted with EtOAc (3×50 mL). The combined organic phase was dried over anhydrous Na₂SO₄. After filtration, the filtrate was concentrated under reduced pressure. The residue was purified by reverse phase flash evaporation chromatography under the following conditions: a column of C18 silica gel column, a mobile phase of methanol-water, a gradient of 40%-70%, an elution time of 10 min, and a detector of UV 254 nm. A grey-white solid of 3,4,5-tris(acetoxy)-6-[3-(2,3-dihydro-1,4-benzodioxin-6-ylmethyl)-4-methylphenyl]oxane-2-y 1 acetate (2.08 g with a purity of 64.5%) was obtained.

The reaction route is as follows:

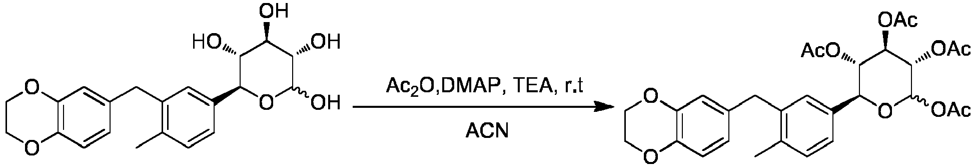

(7) Under stirring, a mixed solution of 4, 5-bis(acetoxy)-2-(carbamoylthio)-6-[3-(2,3-dihydro-1,4-benzodioxin-6-ylmethyl)-4-methyl phenyl]oxane-3-yl acetate (1.55 g, 1.00 eq) and DIPEA (1.80 g, 5.00 eq) in 1,4-dioxane was added with CH₃I (1.20 g, 3.00 eq) for 24 h of reaction at room temperature. The reaction was monitored by LCMS. After the reaction was completed, the reaction was quenched with an ice-water mixture at room temperature. The resulting mixture was extracted with EtOAc (3×50 mL). The combined organic phase was concentrated under reduced pressure. The residue was purified by reverse phase flash evaporation chromatography under the following conditions: a column of C18 silica gel column, a mobile phase of acetonitrile-water, a gradient of 50%-70%, an elution time of 20 min, and a detector of UV 254 nm. A grey-white solid of 4, 5-bis(acetoxy)-6-[3-(2,3-dihydro-1,4-benzodioxin-6-ylmethyl)-4-methylphenyl]-2-(methylt hio)oxy-3-yl acetate (1.09 g with a yield of 71.8%) was obtained.

The reaction route is as follows:

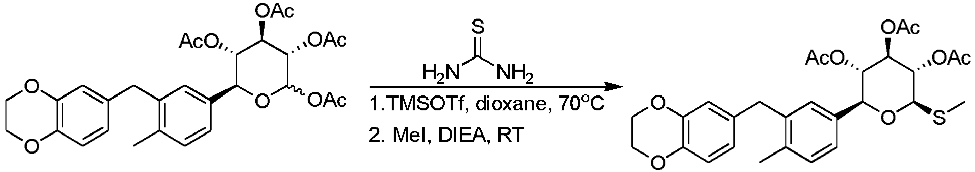

(8) Under stirring, a mixed solution of THF (4 mL), MeOH (8 mL) and water (8 mL) was added with 4,5-bis(acetoxy)-6-[3-(2,3-dihydro-1,4-benzodioxin-6-ylmethyl)-4-methylphenyl]-2-(methylt hio)oxy-3-yl acetate (1.09 g, 2.00 mmol) for 1 h of reaction at room temperature. The mixture was then acidified to pH 2 with saturated NaHSO₄ (aq.). The resulting mixture was extracted with EtOAc (3×50 mL). The combined organic phase was concentrated by distillation under reduced pressure. The residue was purified by reverse phase flash evaporation chromatography under the following conditions: a column of C18 silica gel column, a mobile phase of acetonitrile-water, a gradient of 10%-50%, an elution time of 15 min, and a detector of UV 254 nm. A grey-white solid of 2-[3-(2,3-dihydro-1,4-benzodioxin-6-ylmethyl)-4-methylphenyl]-6-(methylthio)oxane-3,4,5-tr iol (520.1 mg with a yield of 44.6%) was obtained.

The reaction route is as follows: 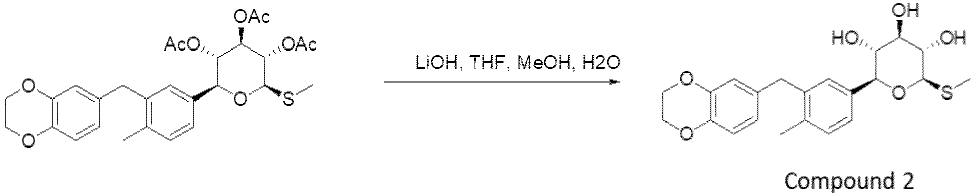 LC-MS: (ES, m/z):[M+23] =441.
¹H NMR:(400 MHz, DMSO-d6) δ 7.09 (s, 3H), 6.77 - 6.70 (m, 1H), 6.62 - 6.56 (m, 2H), 5.19 (d, J = 5.5 Hz, 1H), 5.08 (d, J = 4.5 Hz, 1H), 4.84 (d, J = 5.5 Hz, 1H), 4.33 (d, J = 9.4 Hz, 1H), 4.05 (d, J = 9.1 Hz, 1H), 3.81 (s, 2H), 3.32 (d, J = 1.8 Hz, 1H), 3.31 - 3.13 (m, 2H), 2.17 (s, 3H), 2.05 (d, J = 14.1 Hz, 3H).

### Example 3 Preparation of Compound 3

(1) Under stirring, a mixed solution of 4-ethoxyphenylboronic acid (5.20 g, 31.329 mmol, 1.30 eq) and 5-bromo-2-chlorophenol (5.00 g, 24.102 mmol, 1.00 eq) in dichloromethane (100 mL) was added with Cu(AcO)₂ (4.82 g, 26.512 mmol, 1.1 eq) and pyridine (5.72 g, 72.307 mmol, 1.1 eq) for 2 days of reaction at room temperature. The reaction was quenched with water (100 mL). The resulting mixture was extracted with EtOAc (3×200 mL). The combined organic phase was concentrated under vacuum. The residue was purified by silica gel column chromatography and eluted with PE to obtain a grey-white solid of 4-bromo-1-chloro-2-(4-ethoxyphenoxy)benzene (1.2 g with a yield of 15.20%).

The reaction route is as follows:

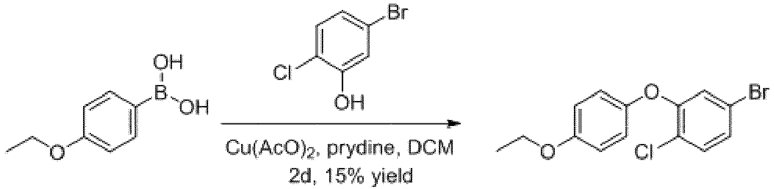

(2) Under a nitrogen atmosphere and stirring, a solution of 4-bromo-1-chloro-2-(4-ethoxyphenoxy)benzene (900.00 mg, 2.747 mmol, 1.00 eq) in THF (15.00 mL) was dropwise added with n-BuLi (2.20 mL, 5.500 mmol, 2.00 eq) at -78°C. After 1 h, the above mixture was dropwise added with a solution of (3R,4S,5R,6R)-3,4,5-tris(benzyloxy)-6-[(benzyloxy)methyl]oxane-2-one (3.70 g, 6.868 mmol, 2.50 eq) in THF (5.00 mL) at -78°C. The reaction system was stirred at -78°C for 1 h of reaction. The reaction system was heated to room temperature and added with saturated NH₄Cl (aq.) to quench the reaction. The resulting mixture was extracted with EtOAc (3 ×200 mL). The combined organic phase was washed with saturated brine (3x100 mL) and dried over anhydrous Na₂SO₄. After filtration, the filtrate was concentrated under reduced pressure, and the residue was purified by reverse phase flash evaporation chromatography under the following conditions: a column of C18 silica gel column, and a mobile phase of FA-water to obtain a grey-white solid of (2R,3R,4S,5R,6R)-3,4,5-tris(benzyloxy)-6-[(benzyloxy)methyl]-2-[4-chloro-3-(4-ethoxyphen oxy)phenyl]oxane-2-ol (1.2 g with a yield of 55.48%).

The reaction route is as follows:

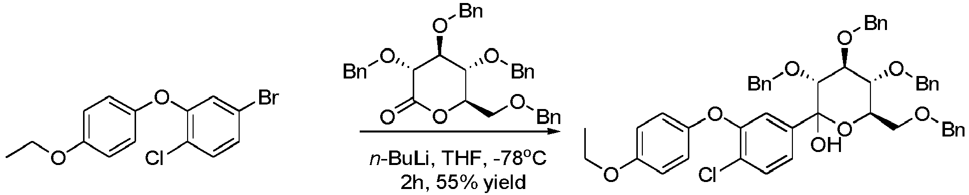

(3) A solution of (2R,3R,4S,5R,6R)-3,4,5-tri(benzyloxy)-6-[(benzyloxy)methyl]-2-[4-chloro-3-(4-ethoxyphenoxy)phenyl]oxane-2-ol (1.25 g, 1.588 mmol, 1.00 eq) in acetonitrile (20 mL) was added into a flask, which was then vacuumized and scoured with nitrogen three times. The system was then cooled to -30°C, and added with triethylsilane (0.69 g, 4.763 mmol, 3.00 eq) and BF₃-Et₂O (0.34 g, 2.381 mmol, 1.50 eq) at -30°C after a few minutes. The reaction system was stirred at -30°C for 1 h, and warmed up naturally to the room temperature. The reaction was quenched with K₂CO₃ (5 mL), and the mixture was extracted with EA (2x200 mL). The combined organic phase was concentrated under reduced pressure. The residue was purified by reverse phase flash evaporation chromatography with the following conditions: a column of C18 silica gel column, a mobile phase of FA-water; and a detector of UV 254 nm. A colorless oil of (2R,3R,4R,5S,6R)-3,4,5-tris(benzyloxy)-2-[(benzyloxy)methyl]-6-[4-chloro-3-(4-ethoxyphen oxy)phenyl]oxane (1.2 g with a yield of 97.99%) was obtained.

The reaction route is as follows:

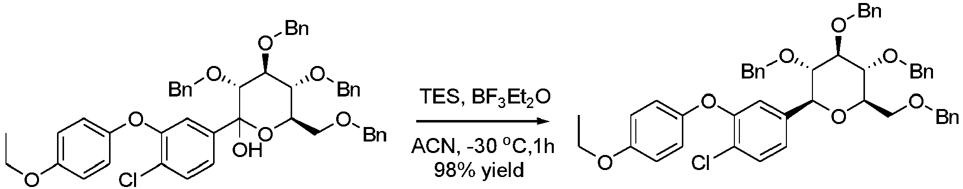

(4) Under stirring, a solution of (2R,3R,4R,5S,6R)-3,4,5-tri(benzyloxy)-2-[(benzyloxy)methyl]-6-[4-chloro-3-(4-ethoxypheno xy)phenyl]oxane (500.00 mg, 0.648 mmol, 1.00 eq) in MeOH (50 mL) was added with Pd/C (150.00 mg, 1.410 mmol, 2.17 eq) at room temperature. Under a hydrogen atmosphere, the mixture was stirred overnight at room temperature under a pressure of 5 atm. The resulting mixture was filtered, and the filter cake was washed with MeOH. The filtrate was concentrated under reduced pressure. The residue was purified by preparative liquid chromatography under the following conditions: a chromatographic column of XBridge Prep OBD C18 column with 19*250 mm and 5 µm, a mobile phase A of water (0.05% NH₃H₂O), a mobile phase B of acetonitrile, a flow rate of 25 mL/min, a gradient of 25 B-40 B, an elution time of 12 min, a UV detector at 254 nm; and RT1 of 11.02. A grey-white solid of (2R,3R,4R,5S,6R)-2-[4-chloro-3-(4-ethoxyphenoxy)phenyl]-6-(hydroxymethyl)oxane-3,4,5-tr iol (92 mg with a yield of 34.03%) was obtained.

The reaction route is as follows: 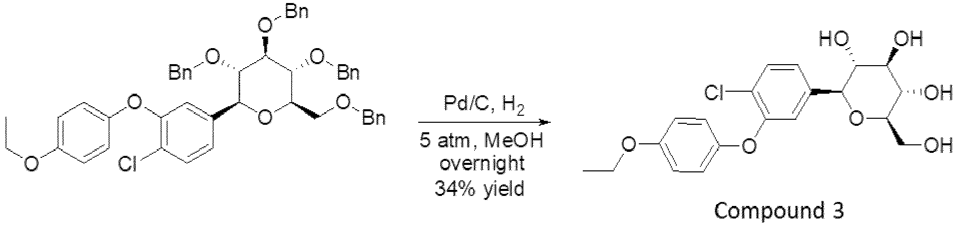 LC-MS 3: (ES, m/z): [M-1]=409
H-NMR 3: ¹H NMR (400 MHz, Methanol-*d*₄) δ 7.42 (d, *J* = 8.2 Hz, 1H), 7.17 (dd, *J* = 8.2, 1.9 Hz, 1H), 7.00 (d, *J* = 2.0 Hz, 1H), 6.89 (s, 4H), 4.11 - 3.95 (m, 3H), 3.85 (d, *J* = 11.9 Hz, 1H), 3.71 - 3.54 (m, 1H), 3.56 - 3.34 (m, 3H), 3.21 (t, *J* = 9.0 Hz, 1H), 1.38 (t, *J* = 7.0 Hz, 3H).

The present invention further adopted similar methods to prepare the following compounds having structural formula and mass spectrum data as shown in the following table:

| Number | Compound structural formula | MS: m/z [M-H]⁻ |
|---|---|---|
| 4 | 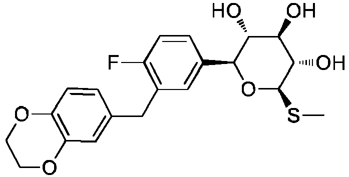 | 421 |
| 5 | 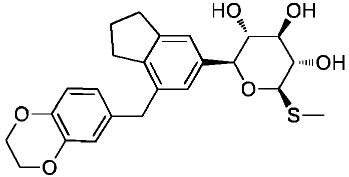 | 443 |
| 6 | 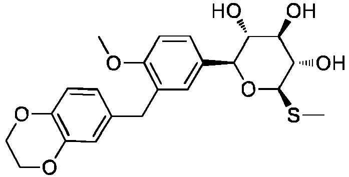 | 433 |
| 7 | 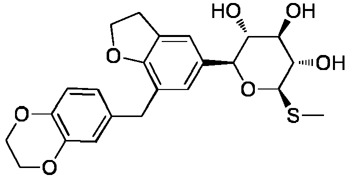 | 445 |
| 8 | 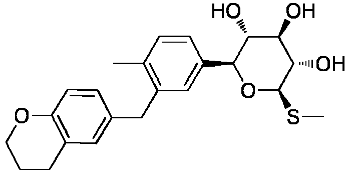 | 415 |
| 9 | 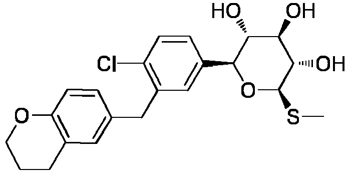 | 435 |
| 10 | 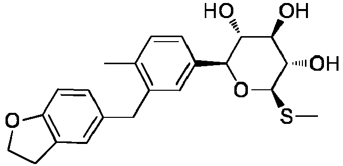 | 401 |
| 11 | 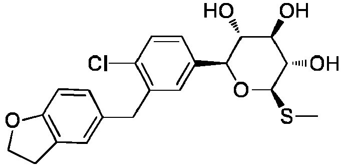 | 421 |
| 12 | 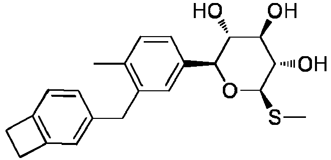 | 385 |
| 13 | 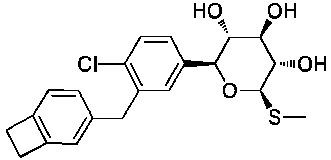 | 405 |
| 14 | 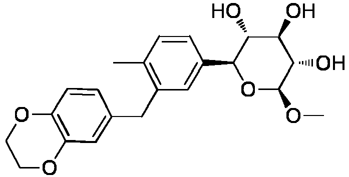 | 401 |
| 15 | 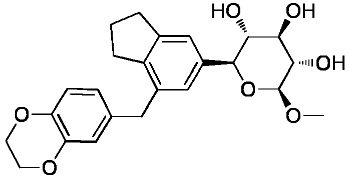 | 427 |
| 16 | 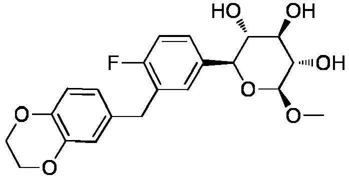 | 405 |
| 17 | 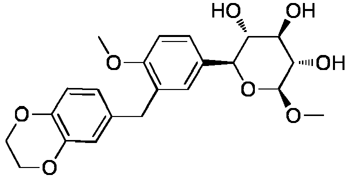 | 417 |
| 18 | 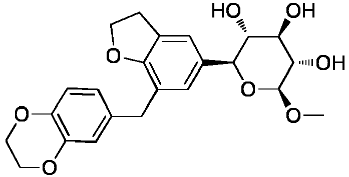 | 429 |
| 19 | 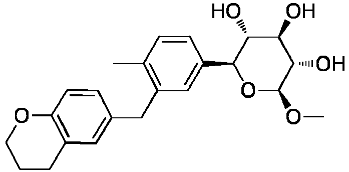 | 399 |
| 20 | 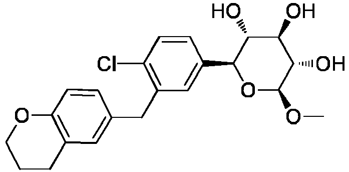 | 419 |
| 21 | 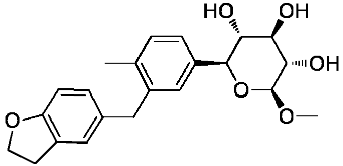 | 385 |
| 22 | 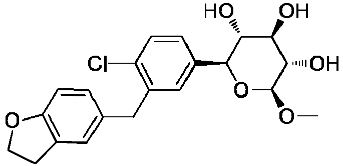 | 405 |
| 23 | 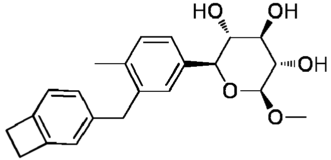 | 369 |
| 24 | 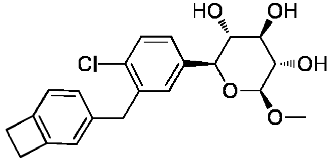 | 389 |
| 25 | 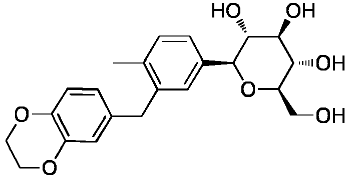 | 401 |
| 26 | 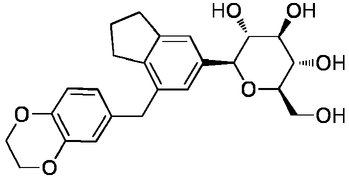 | 427 |
| 27 | 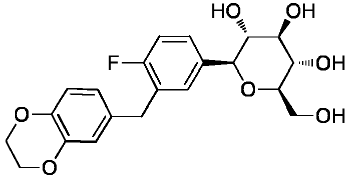 | 405 |
| 28 | 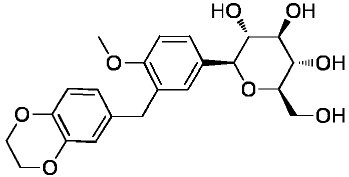 | 417 |
| 29 | 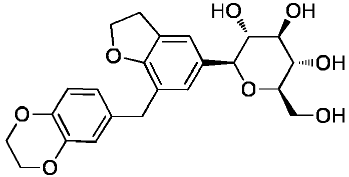 | 429 |
| 30 | 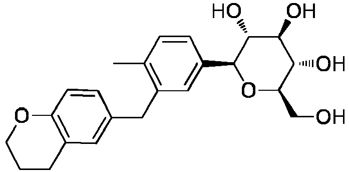 | 399 |
| 31 | 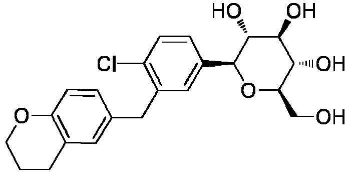 | 419 |
| 32 | 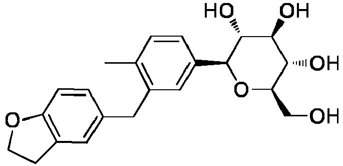 | 385 |
| 33 | 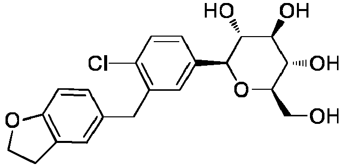 | 405 |
| 34 | 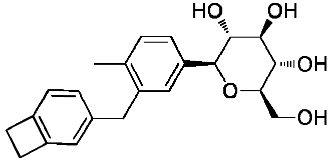 | 369 |
| 35 | 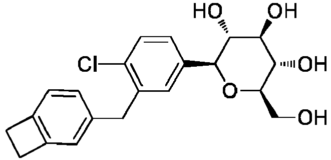 | 389 |

In order to evaluate the efficacy of the glycoside derivatives of the present invention, the following biological activity examples were carried out.

### Biological activity examples:

### Test Example 1-In vitro SGLT2 inhibitory activity

The effect of the compounds on SGLT2-mediated glucose uptake was investigated by establishing an SGLT2 overexpression cell model and using the fluorescent glucose (2-deoxy-1-[(7-nitro-2,1,3-benzoxadiazol-4-yl)amino]-D-glucose, 1-NBDG) tracer method to screen out candidate compounds with SGLT2 inhibitory activity.

### 1. Materials and methods

### (1) Materials

The humanized SGLT2 overexpression cells (CHO-SGLT2) constructed from Chinese hamster ovary (CHO) cells were cultured in 1640 medium (Gibco) containing 10% fetal bovine serum (Gibco, 10270), subcultured at a ratio of 1:3, and replaced with new culture medium 3 times every week. Fluorescence detection cell culture plates and other culture flasks were purchased from Corning Company. Dapagliflozin was used as a positive control, and the test compounds were compound 2 and compound 3. Appropriate amounts of all compounds were weighed, prepared into 100 mM stock solutions with DMSO, and stored at 4°C in the dark.

### (2) Main reagents

Preparation of choline buffer: 140 mM choline chloride, 5 mM KCl, 2.5 mM CaCl₂, 1 mM MgSO₄, 1 mM KH₂PO₄, 10 mM HEPES, pH adjusted to 7.4 with Tris base. The filtration was conducted with a 0.22 µm membrane.

Preparation of Na⁺ sodium buffer: 140 mM NaCl, 5 mM KCl, 2.5 mM CaCl₂, 1 mM MgSO₄, 1 mM KH₂PO₄, 10 mM HEPES, pH adjusted to 7.4 with Tris base.

Preparation of neutral lysate: 1% Nonidet P-40, 1% sodium deoxycholate, 40 mM KCl, pH adjusted to 20 mM Tris base.

### (3) Instruments

### Multifunctional microplate reader Biotek Synergy2

### (4) Detection of uptake of NBDG by cells:

Before the 1-NBDG uptake test, CHO-SGLT2 cells were digested and seeded in a 96-well cell culture plate with a cell number of 40,000/well. After the cells were cultured for 48 h, the medium was discarded, and 100 µl of choline buffer was added to each well. After the cells were starved at 37°C for 30 min, the choline buffer was discarded and 100 µl of the newly prepared Na⁺ sodium buffer containing 100 µM 1-NBDG and the test compounds (Compound) was added. The solvent DMSO was used as the negative control (Control) for the test compounds. The Na⁺ sodium buffer without 1-NBDG was used as the blank control (Blank). After 1 h of culture, the incubation solution was discarded. The cells were washed with choline buffer solution 3 times, and then 50 µl of neutral lysate was added to each well to lyse the cells on ice for 10 min. The fluorescence value of each well was detected using a multi-functional microplate reader at Em=485/20 nm and Ex=528/20 nm.

### (5) Calculation and statistical methods

At least 3 experimental replicates were set for each compound at indicated concentrations, and the data were expressed as mean±SEM. Uptake amount of 1-NBDG by cells= fluorescence value_{1-NBDG}-fluorescence value_{Blank}. Inhibition rate on uptake amount of SGLT2 glucose (%)= (uptake amountcontrol - uptake amount_{compound})/uptake amount_{control}×100%. Concentration-inhibition rate curves were plotted using Graphpad Prism software, and IC₅₀ values were calculated accordingly.

### 2. Results

According to the pre-experiment results of each compound, the stock solution was diluted 10/20 times, and several concentration gradients were set (see Table 1). The effects of the compounds at different concentrations on the uptake of 1-NBDG by cells were detected, and the results were shown as the SGLT2 inhibition rate of each compound at a specific concentration (Table 1). Concentration-inhibition rate curve of each compound was plotted (FIG. 4). Taking the maximum inhibitory activity of the positive control dapagliflozin as 100%, the relative maximum activity (Max%) of each compound and the IC₅₀ value of the compounds inhibiting SGLT2 were calculated (Table 2).

**Table 1 Inhibition rate of compounds on SGLT2 (%)**

| Compound concentration (mol/L) | 2 | 3 | Dapagliflozin |
|---|---|---|---|
| 1 × 10⁻¹² | 0.3±6.4 | | 6.8±5.9 |
| 5 × 10⁻¹² | | | |
| 1 × 10⁻¹¹ | 0.0±5.1 | | 1.9±9.2 |
| 5 × 10⁻¹¹ | 39.2±2.1 | 3.7±4.2 | 42.0±2.8 |
| 1 × 10⁻¹⁰ | 40.6±1.0 | | |
| 5 × 10⁻¹⁰ | 61.9±3.6 | 8.7±2.1 | 60.1±2.0 |
| 1×10⁻⁹ | | | |
| 5×10⁻⁹ | 80.7±3.0 | 42.8±2.3 | 84.8±3.2 |
| 1×10⁻⁸ | | | |
| 5×10⁻⁸ | | 71.7±1.7 | 84.3±4.0 |
| 1×10⁻⁷ | | | |
| 5×10⁻⁷ | 81.6±2.5 | 78.7±1.9 | |
| 1×10⁻⁶ | 106.8±1.1 | | |
| 5×10⁻⁶ | | | |
| 1×10⁻⁵ | | 96.4±8.4 | 106.4±4.2 |

**Table 2. Inhibitory activity and IC₅₀ of compounds of the present invention inhibiting SGLT2**

| Compound No. | Compound structural formula | IC50 (nmol/1) |
|---|---|---|
| 2 | 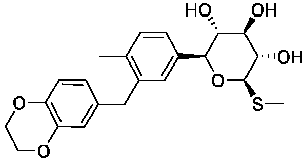 | 0.105 |
| 3 | 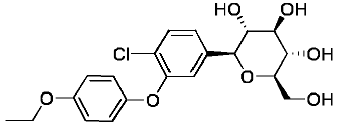 | 6.745 |
| 5 | 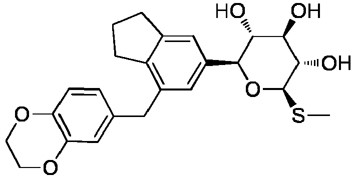 | 2.260 |
| 8 | 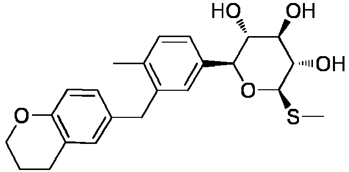 | 0.356 |
| 11 | 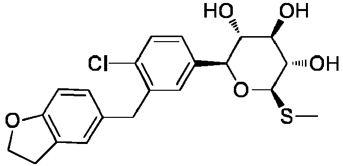 | 3.521 |
| 12 | 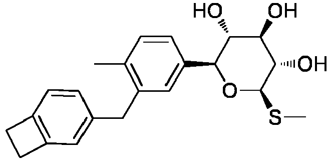 | 1.012 |
| 24 | 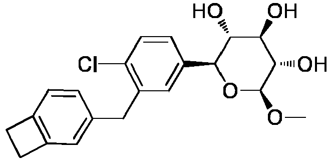 | 1.362 |
| 27 | 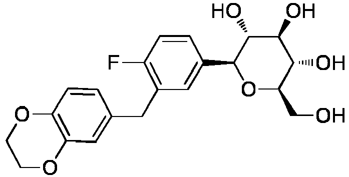 | 3.321 |
| Control Dapagliflozin | | 0.147 |

According to the above results, it can be seen that the compounds of the present invention have good inhibitory activity against SGLT2.

### Test Example 2 Evaluation of the in vivo activity of the compounds of the present invention: the effect on the blood glucose level of normal mice after oral administration of glucose

### 1. Materials and methods

### (1) Animals

ICR mice (male, 18-20 g), purchased from Beijing Vital River Laboratories Co., Ltd., were fed adaptively for 4 days, and randomly divided into 4 groups, namely normal control group, positive control group, and two groups of test compounds, with 8 mice in each group.

### (2) Compound and dose setting

Compound 2 of the present invention was taken as an example, and two doses, 2 mg/kg and 4 mg/kg, were used for the animal experiments. The positive control drug dapagliflozin was used at a dose of 2 mg/kg. The aforementioned stock solutions were diluted to appropriate concentrations with double distilled water.

### (3) Experimental design

The mice were fasted overnight. The normal control group was administered with 0.5% DMSO in double distilled water (note: the stock solution was prepared with DMSO) by gavage, the positive control group was administered with dapagliflozin at 2 mg/kg by gavage, and the two groups of test compounds were administered with Compound 2 at 2 mg/kg and 4 mg/kg respectively by gavage. After 1 h, blood was collected at 0 min (fasting), and then mice in each group were administered with glucose (2.0 g/kg) solution by gavage. Blood was collected at 30 min, 60 min, and 120 min after glucose administration. The blood glucose level was measured using glucose oxidase-peroxidase method, and the area under the blood glucose curve was calculated.

### (4) Statistical methods

The data were expressed as mean±SD. The comparison between multiple groups was performed by one-way analysis of variance, and the comparison between two groups was performed by Dunnett's t test. Statistical graphing was performed using Graphpad Prism software.

The results are shown in FIG. 5 and Table 3.

FIG. 5 shows the effect of Compound 2 on the blood glucose curve and the area under the blood glucose curve of normal ICR mice after oral administration of glucose. ***p<0.001, vs. Nor group.

**Table 3. Effect of Compound 2 on blood glucose level of normal mice after oral administration of glucose (mean±SD)**

| Group | Dose (mg/kg) | Blood glucose (mg/dl) | | | | AUC (mg/dl·h) |
|---|---|---|---|---|---|---|
| | | 0 min | 30 min | 60 min | 120 min | |
| Normal control group | - | 106.8±9.0 | 292.0±47.1 | 221.5±34.6 | 117.4±7.4 | 397.5±49.1 |
| Dapagliflozin | 2 | 71.8±6.6*** | 213.2±26.6*** | 122.2±16.1^{∗∗∗} | 83.0±13.5*** | 257.7±24.0*** |
| Compound 2 | 2 | 75.9±6.7*** | 207.8±13.6*** | 119.3±12.5*** | 73.0±11.4*** | 248.8±18.0*** |
| Compound 2 | 4 | 63.6±8.3*** | 189.5±14.3*** | 103.2±5.1^{∗∗∗} | 78.2±13.9*** | 227.1±13.1*** |

Compared with the normal control group (Nor), the positive control drug dapagliflozin not only lowered the fasting blood glucose (P<0.001), but also significantly lowered the blood glucose level at each time point after the glucose administration (P<0.001), and reduced the area under the curve (AUC) of blood glucose by 35.2% (P<0.001). The test Compound 2 at two doses of 2 mg/kg and 4 mg/kg can lower the fasting blood glucose (P<0.001), significantly lower the blood glucose level at each time point after the glucose administration (P<0.001), and significantly reduce the area under the curve (AUC) of blood glucose (P<0.001) by 37.4% and 42.9% respectively, which was even better than the positive control drug dapagliflozin.

### Test Example 3 Research on efficacy of the compounds of the present invention in in vivo anti-Alloxan diabetes model mice

### 1. Materials and methods

### (1) Animals

Alloxan diabetes mice were used. Normal ICR mice (male, 22-24 g) were randomly divided into groups including normal group, model group (con), positive control group and test compound groups with four different doses (n=11), and the mice without injection of Alloxan were in a normal control group (n=10).

### (2) Test compound and dose setting

Compound 2 of the present invention was taken as an example, and 4 doses of 0.25 mg/kg, 0.5 mg/kg, 1.0 mg/kg and 2.0 mg/kg were used. The positive control drug dapagliflozin was used at a dose of 2.0 mg/kg.

### (3) Experimental design

Mice were administered with drugs by gavage. The positive control group was administered with dapagliflozin (2.0 mg/kg), and the test groups were administered with different doses of Compound 2 (0.25 mg/kg, 0.5 mg/kg, 1.0 mg/kg and 2.0 mg/kg).

Single administration: Mice were fasted for 3 h, and then the fasting blood (0 min) was collected. Then the mice in each group were administered with drugs by gavage. After 1 h, they were administered with a glucose (2.0 mg/kg) solution. Blood was collected at 30 min, 60 min, 120 min, and 180 min after the glucose administration. The blood glucose level was measured using glucose oxidase-peroxidase method, and the area under the blood glucose curve was calculated.

On the 6th day of continuous administration, under the condition of no fasting, blood samples were collected from mice before administration (0 h), 1 h, 2 h, 4 h, 8 h and 24 h after administration to measure blood glucose.

On the 9th day of continuous administration, the mice were fasted for 1 h and then administered with drugs by gavage. An oral glucose tolerance test (OGTT) was performed 1 h after administration.

On the 16th day of continuous administration, under the condition of no fasting, urine samples were collected from mice 1 h after administration to measure the concentration of urine glucose.

On the 22nd day of continuous administration, the mice were fasted for 1 h and then administered with drugs by gavage, and blood samples were collected to measure the fasting blood glucose 1 h after administration.

On the 28th day of continuous administration, under the condition of no fasting, blood samples were collected from mice 1 h after administration to measure the random blood glucose.

On the 32nd day of continuous administration, under the condition of no fasting, blood samples were collected from mice to measure glycosylated hemoglobin (HbA1c).

### (4) Statistical methods

The data were expressed as mean±SEM (n=10-11). The comparison between multiple groups was performed by one-way analysis of variance, and the comparison between two groups was performed by Dunnett's t test. Statistical graphing was performed using Graphpad Prism software.

### 2. Results

(1) A single administration of Compound 2 (0.25 mg/kg, 0.5 mg/kg, 1.0 mg/kg and 2.0 mg/kg) can significantly lower the blood glucose level and reduce the area under the curve (AUC) of blood glucose in Alloxan diabetic mice after glucose administration. At equal doses, Compound 2 had stronger efficacy than that of the positive control drug.
(2) Continuous administration of Compound 2 (0.25 mg/kg, 0.5 mg/kg, 1.0 mg/kg and 2.0 mg/kg) for 6 days can significantly lower the non-fasting blood glucose level of Alloxan diabetic mice (p<0.001 or p<0.01), reduced the area under the curve (AUC) of blood glucose, and improved oral glucose tolerance. Dapagliflozin (2.0 mg/kg) showed significantly weakened hypoglycemic effect after 8 h and no significant effect after 24 h, while Compound 2 (0.25 mg/kg, 0.5 mg/kg, 1.0 mg/kg and 2.0 mg/kg) still had a good hypoglycemic effect after 8-24 h. Compared with dapagliflozin (2.0 mg/kg), Compound 2 (0.25 mg/kg, 0.5 mg/kg, 1.0 mg/kg and 2.0 mg/kg) had a significantly longer duration of drug effect than that of the positive control drug dapagliflozin (p<0.05, p <0.01 or p<0.001).
(3) Continuous administration of Compound 2 (0.25 mg/kg, 0.5 mg/kg, 1.0 mg/kg and 2.0 mg/kg), on the 9th and 22nd day, can significantly lower fasting blood glucose level in Alloxan diabetic mice (p<0.001). Compared with the positive control drug dapagliflozin (2.0 mg/kg), Compound 2 (1.0 mg/kg and 2.0 mg/kg) showed a more significant reduction in blood glucose level (p <0.01), and had a significantly stronger efficacy than that of the positive control drug.
(4) Continuous administration of Compound 2 (0.25 mg/kg, 0.5 mg/kg, 1.0 mg/kg and 2.0 mg/kg) for 28 days can significantly lower the random blood glucose level in Alloxan diabetic mice (p<0.001). Compared with the positive control drug dapagliflozin (2.0 mg/kg), Compound 2 (0.5 mg/kg, 1.0 mg/kg and 2.0 mg/kg) showed a more significant reduction in blood glucose level (p<0.05, p<0.01). Continuous administration of Compound 2 (0.5 mg/kg, 1.0 mg/kg and 2.0 mg/kg) for 32 days significantly lowered the level of glycosylated hemoglobin in Alloxan diabetic mice, and compared with the positive control drug dapagliflozin (2.0 mg/kg), Compound 2 (0.5 mg/kg, 1.0 mg/kg and 2.0 mg/kg) showed significant reduction in the glycosylated hemoglobin (p<0.05) on the 32nd day, indicating a significantly stronger efficacy than that of the positive control drug.
(5) Continuous administration of Compound 2 (0.25 mg/kg, 0.5 mg/kg, 1.0 mg/kg and 2.0 mg/kg) for 16 days can significantly increase the concentration of urine glucose in Alloxan diabetic mice, indicating a significantly stronger efficacy than that of the positive control drug.

The test results are shown in Table 4-Table 7 below.

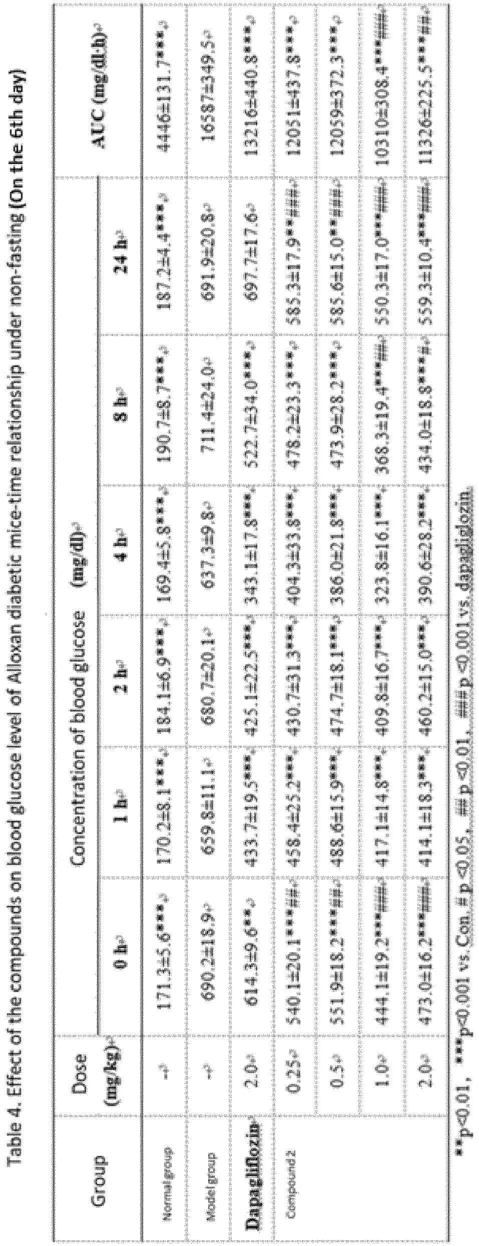

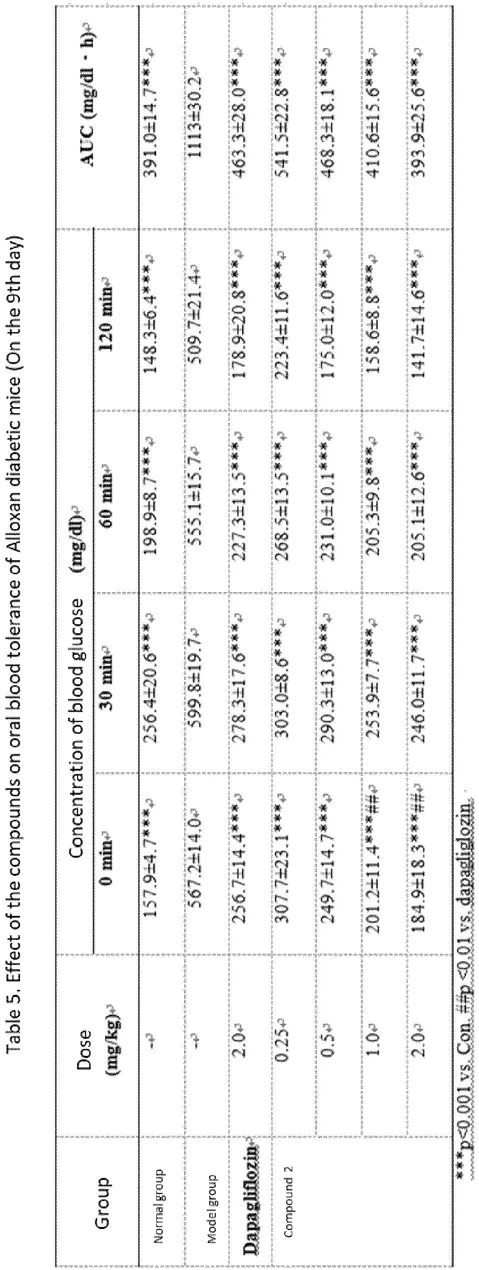

**Table 6. Effect of the compounds on lowering fasting blood glucose in Alloxan mice (on the 9th day and 22nd day)**

| Group | Dose (mg/kg) | Concentration of fasting blood glucose (mg/dl) | |
|---|---|---|---|
| | | The 9th day | The 22nd day |
| Normal group | - | 157.9±4.7^{∗∗∗} | 173.6±6.4*** |
| Model group | - | 567.2±14.0 | 667.5±20.3 |
| Dapagliflozin | 2.0 | 256.7±14.4^{∗∗∗} | 316.5±18.6^{∗∗∗} |
| Compound 2 | 0.25 | 307.7±23.1^{∗∗∗} | 305.6±16.5^{∗∗∗} |
| | 0.5 | 249.7±14.7*** | 236.0±13.7***## |
| | 1.0 | 201.2±11.4^{∗∗∗}## | 224.4±17.6^{∗∗∗}## |
| | 2.0 | 184.9±18.3^{∗∗∗}## | 211.9±17.8^{∗∗∗}## |

| | | | |
|---|---|---|---|
| ***p<0.001 vs. model group ## p<0.01 vs. dapagliflozin. | | | |

**Table 7. Effect of the compounds on random blood glucose (on the 28th day) and glycosylated hemoglobin (on the 32nd day) in Alloxan mice**

| Group | Dose (mg/kg) | Concentration of blood glucose (mg/dl) | HbAlc (%) |
|---|---|---|---|
| Normal group | - | 150.4±5.1*** | 2.2±0.2^{∗∗∗} |
| Model group | - | 736.3±28.5 | 7.8±0.6 |
| Dapagliflozin | 2.0 | 499.3±13.0*** | 6.6±0.5 |
| Compound 2 | 0.25 | 491.1±26.5*** | 7.2±0.4 |
| | 0.5 | 437.9±23.9^{∗∗∗}# | 6.1±0.4^{∗} |
| | 1.0 | 395.8±24.1^{∗∗∗}## | 5.9±0.6^{∗} |
| | 2.0 | 391.2±26.0^{∗∗∗}## | 6.0±0.4^{∗} |

| | | | |
|---|---|---|---|
| ^{∗}p<0.05,^{∗∗∗}p<0.001 vs. Model group # p<0.05,## p<0.01 vs. dapagliflozin. | | | |

In addition, continuous administration of Compound 2 (0.25 mg/kg, 0.5 mg/kg, 1.0 mg/kg and 2.0 mg/kg) for 22 days had no obvious effect on the body weight, food intake and water intake of Alloxan diabetic mice, indicating a good safety.

### Test example 4 Pharmacodynamic effect of the compounds of the present invention on spontaneous type 2 diabetes mellitus db/db mice

### 1. Materials and methods

### (1) Animals

Spontaneous type 2 diabetes mellitus db/db mice and the normal control db/m mice (male, 5-6 weeks old) were used. The db/db mice were randomly divide into 6 groups based on random blood glucose, fasting blood glucose, percentage of blood glucose reduction at 40 min, blood triglycerides, total blood cholesterol, and body weight, which were model group (con), positive control group and three groups of test compound with different doses (n=10).

### (2) Compound and dose setting

Compound 2 of the present invention was taken as an example, and three doses of 0.5 mg/kg, 1.0 mg/kg and 2.0 mg/kg were used. The positive control drug dapagliflozin was used at a dose of 2.0 mg/kg.

### (3) Experimental design

Mice were administered with drugs by gavage. The positive control group was administered with dapagliflozin (2.0 mg/kg), and the three groups of Compound 2 were administered with 0.5 mg/kg, 1.0 mg/kg and 2.0 mg/kg of Compound 2, respectively.

The mice were fasted for 2 h, and then the 0 h (fasting) blood samples were collected. Then the mice in each group were administered with drugs by gavage, and blood samples were collected at 1 h, 2 h, 4 h, 6 h, 8 h and 24 h after administration. The blood glucose level was measured using glucose oxidase-peroxidase method, and the area under the blood glucose curve was calculated. Moreover, urine samples were collected 2 h and 4 h after administration to determine the concentration of urine glucose.

On the 15th day of continuous administration, before the mice were fasted, blood samples were collected to measure the random blood glucose. Then the mice were fasted and administered with the compounds, and after 2 h, blood samples were collected to measure the fasting blood glucose.

On the 22nd day of continuous administration, the mice were fasted and administered with the compounds, and after 2 h, blood samples were collected to measure the blood glucose, blood triglyceride and total cholesterol.

On the 28th day of continuous administration, the mice were not fasted, and blood samples were collected to measure the random blood glucose and glycosylated hemoglobin (HbA1c), respectively.

### (4) Statistical methods

The data were expressed as mean±SEM (n=10). The comparison between multiple groups was performed by one-way analysis of variance, and the comparison between two groups was performed by Dunnett's t test. Statistical graphing was performed using Graphpad Prism software.

### 2. Results

(1) A single administration of Compound 2 of the present invention (0.5 mg/kg, 1.0 mg/kg and 2.0 mg/kg) can significantly lower the fasting blood glucose level of spontaneous type 2 diabetes mellitus db/db mice at 1 h, 2 h, 4 h, 6 h, 8 h and 24 h after administration (p<0.001 or p<0.01), and significantly reduce the area under the curve (AUC) of blood glucose. Compared with the positive control drug dapagliflozin (2.0 mg/kg), Compound 2 (0.5 mg/kg, 1.0 mg/kg and 2.0 mg/kg) had significantly stronger efficacy than that of the positive control drug (p<0.01 or p<0.001). A single administration of Compound 2 (0.5 mg/kg, 1.0 mg/kg and 2.0 mg/kg) can lower the blood glucose level of spontaneous type 2 diabetes mellitus db/db mice for up to 24 h, which was significantly longer than that of the positive control drug dapagliflozin (2.0 mg/kg).
(2) 2 h after the single administration of Compound 2 of the present invention (0.5 mg/kg, 1.0 mg/kg and 2.0 mg/kg), the concentration of urine glucose of db/db mice was increased. 4 h after the administration, the concentration of urine glucose of db/db mice was not increased. Compound 2 had the same effect as the positive control drug dapagliflozin.
(3) Continuous administrations of Compound 2 of the present invention with different doses (0.5 mg/kg, 1.0 mg/kg and 2.0 mg/kg) for multiple times can all significantly lower fasting blood glucose and random blood glucose of spontaneous type 2 diabetes mellitus db/db mice. At equal doses, Compound 2 (2.0 mg/kg) had significantly stronger efficacy than that of the positive control drug dapagliflozin (2.0 mg/kg) (p<0.001, p<0.01 or p<0.05).
(4) On the 22nd day of the continuous administration of Compound 2 of the present invention (0.5 mg/kg, 1.0 mg/kg and 2.0 mg/kg), different doses of Compound 2 can all significantly lower the blood triglycerides (TG) level in spontaneous type 2 diabetes mellitus db/db mice, and had no effect on the concentration of blood cholesterol. There was no difference between Compound 2 of the present invention and the positive control drug dapagliflozin.
(5) On the 28th day of the continuous administration of Compound 2 of the present invention (0.5 mg/kg, 1.0 mg/kg and 2.0 mg/kg), different doses of Compound 2 can all obviously lower the glycosylated hemoglobin level of spontaneous type 2 diabetes db/db mice. At equal doses, Compound 2 (2.0 mg/kg) of the present invention had stronger efficacy than that of the positive control drug dapagliflozin (2.0 mg/kg).
(6) Continuous administrations of Compound 2 of the present invention (0.5 mg/kg, 1.0 mg/kg and 2.0 mg/kg) had no significant effect on the body weight, food intake and water intake of the spontaneous type 2 diabetes mellitus db/db mice.

The results are shown in Tables 8-11 below.

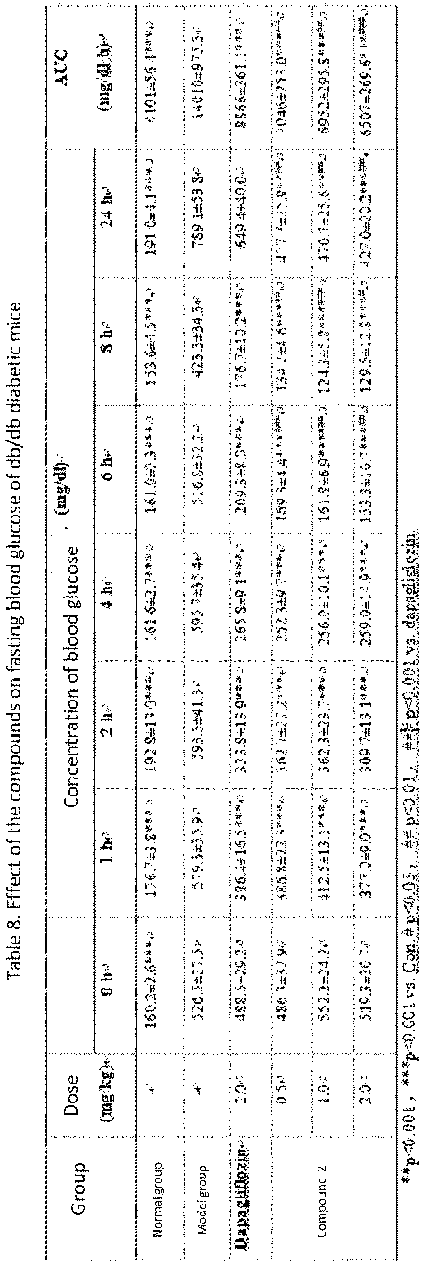

**Table 9. Effect of the compounds on fasting blood glucose of db/db mice (on the 15th day and 22nd day)**

| Group | Dose (mg/kg) | Fasting blood glucose (mg/dl) | |
|---|---|---|---|
| | | The 15th day | The 22nd day |
| Normal group | - | 96.3±3.2^{∗∗∗} | 85.6±2.6^{∗∗∗} |
| Model group | - | 457.6±25.0 | 443.1±23.6 |
| Dapagliflozin | 2.0 | 183.0±11.2^{∗∗∗} | 191.8±11.0^{∗∗∗} |
| Compound 2 | 0.5 | 185.0±13.6^{∗∗∗} | 193.8±14.7^{∗∗∗} |
| | 1.0 | 197.9±11.2^{∗∗∗} | 204.1±12.2^{∗∗∗} |
| | 2.0 | 162.7±12.1^{∗∗∗} | 159.5±8.5^{∗∗∗#} |

| | | | |
|---|---|---|---|
| ^{∗∗∗}p<0.001 vs. Model group | | | |

**Table 10. Effect of the compounds on random blood glucose of db/db mice (on the 15th day and 28th day)**

| Group | Dose (mg/kg) | Blood glucose (mg/dl) | |
|---|---|---|---|
| | | The 15th day | The 22nd day |
| Normal group | - | 128.7±4.3^{∗∗∗} | 111.7±3.9^{∗∗∗} |
| Model group | - | 572.7±29.1 | 452.4±17.6 |
| Dapagliflozin | 2.0 | 344.7±24.1^{∗∗∗} | 317.0±16.4^{∗∗∗} |
| Compound 2 | 0.5 | 307.9±18.4^{∗∗∗} | 272.4±16.5^{∗∗∗} |
| | 1.0 | 322.7±11.1^{∗∗∗} | 258.4±12.8^{∗∗∗#} |
| | 2.0 | 240.4±16.4^{∗∗∗##} | 194.7±14.0^{∗∗∗###} |

| | | | |
|---|---|---|---|
| ^{∗∗∗}p<0.001 vs. Model group. # p<0.05, ##p<0.01, ###p<0.001 vs. dapagliflozin. | | | |

**Table 11. Effect of the compounds on glycosylated hemoglobin of db/db mice (on the 28th day)**

| Group | Dose | HbAlc(%) |
|---|---|---|
| Normal group | - | 3.3±0.1^{∗∗∗} |
| Model group | - | 6.6±0.4 |
| Dapagliflozin | 2.0 | 5.1±0.3^{∗∗} |
| Compound 2 | 0.5 | 47±03^{∗∗∗} |
| | 1.0 | 4.8±0.2^{∗∗} |
| | 2.0 | 4.0±0.2^{∗∗∗##} |

| | | |
|---|---|---|
| ^{∗∗}p<0.01, ^{∗∗∗}p<0.001 vs. Model group ## p<0.01 vs. dapagliflozin. | | |

It can be seen from the above results that Compound 2 of the present invention had a particularly favorable effect on spontaneous type 2 diabetes mellitus mice, including a longer duration of hypoglycemic effect than that of the positive control drug dapagliflozin, a stronger efficacy of lowering fasting blood glucose and random blood glucose than that of the positive control drug, and a stronger effect of lowering the level of glycosylated hemoglobin than that of the positive control drug. The above results show that the compounds of the present invention had excellent pharmacodynamic effect and good safety.

### Test Example 5 Research on safety of the compounds of the present invention

The compounds of the present invention were subjected to the following acute toxicity studies in mice.

### 1. Purpose of the test

Compound 2 of the present invention was taken as an example, and its acute toxicity in mice was compared with that of dapagliflozin.

### 2. Dose setting

Mice were administered with Compound 2 or dapagliflozin at 3000 mg/kg once by gavage, and the acute toxicity difference between Compound 2 and dapagliflozin was compared.

### 3. Measurement frequency and method of indicators

### 3.1 General state observation and death rate

After administration, the animals were observed for 7 consecutive days, once a day beside the cage, and closely observed particularly 4 h after the administration. The symptoms of toxicity reaction and death time were recorded.

### 3.2 Test grouping

The mice were randomly divided into 3 groups according to body weight, which were blank control group, dapagliflozin group and Compound 2 group, with 10 mice in each group, half male and half female. On the day before administration, the animals were fasted but provided with water for 17 h. Each test drug group was administered with the corresponding test drug once by gavage, and the blank control group was administered with the same dose of vehicle once by gavage.

### 4. Experimental results

The mice in the Compound 2 group and dapagliflozin group were administered with the test solutions at the same dose of 3000 mg/kg by gavage. The Compound 2 group had a significantly lower toxicity than that of the dapagliflozin group. There was a significant difference in the death rate between genders of the two groups, where the dead mice were all male, and none of the female mice died.

**Table 1 Summary of death time and death rate**

| Group | Gender | Number (N) | D1 | D2 | D3 | D4 | D5 | D6 | D7 | Death rate (%) |
|---|---|---|---|---|---|---|---|---|---|---|
| Blank | F | 5 | 0 | 0 | 0 | 0 | 0 | 0 | 0 | 0 |
| | M | 5 | 0 | 0 | 0 | 0 | 0 | 0 | 0 | 0 |
| Dapagliflozin | F | 5 | 0 | 0 | 0 | 0 | 0 | 0 | 0 | 0 |
| | M | 5 | 0 | 0 | 1 | 1 | 0 | 2 | 0 | 80 |
| Compound 2 | F | 5 | 0 | 0 | 0 | 0 | 0 | 0 | 0 | 0 |
| | M | 5 | 0 | 0 | 0 | 1 | 0 | 1 | 0 | 40 |

| | | | | | | | | | | |
|---|---|---|---|---|---|---|---|---|---|---|
| Note: "F" represents female; "M" represents male; and "D1" represents day 1. | | | | | | | | | | |

The descriptions of the above examples are only used to help understand the method and core idea of the present invention. It should be noted that for those of ordinary skill in the art, without departing from the principle of the present invention, several improvements and modifications can be made to the present invention, and these improvements and modifications also fall within the scope of the claims of the present invention.

## Claims

1. A glycoside derivative, which is a compound represented by formula I or a pharmaceutically acceptable salt thereof: 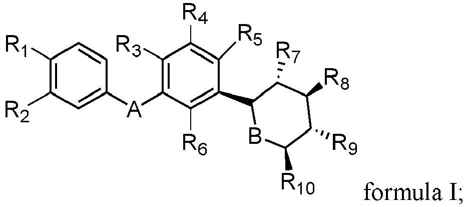
wherein, A is an oxygen atom, -(CH₂)ₘ- or -NH-; m is 1, 2 or 3;
B is an oxygen atom or a sulfur atom;
R₁, R₂, R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
alternatively, R₁ and R₂ together form heterocyclyl, cycloalkyl, aryl or heteroaryl fused to a benzene ring, and/or R₃ and R₄ together form cyclopentyl or oxacyclopentyl fused to a benzene ring;
R₇, R₈, R₉, and R₁₀ are independently selected from the group consisting of hydrogen, hydroxyl, alkyl, alkoxy and alkylthio; and
R₁₁ and R₁₁ₐ are independently selected from the group consisting of a hydrogen atom and alkyl;
wherein, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio are unsubstituted or further substituted by one or more substituents selected from the group consisting of halogen, hydroxyl, amino, carboxyl, cyano, alkyl, alkoxy and nitro.

2. The glycoside derivative according to claim 1, wherein R₁, R₂, R₃, R₄, R₅ and R₆ are independently selected from the group consisting of hydrogen, hydroxyl, carboxyl, halogen, -CN, C1∼C6 alkyl, C1∼C6 alkoxy, C2∼C16 alkoxyalkoxy, C3∼C6 cycloalkyl, C6∼C12 aryl, C2∼C12 heteroaryl, -O-C6∼C12 aryl, -O-C2∼C12 heteroaryl, -OCH₂-C6∼C12 aryl, -OCH₂-C2∼C12 heteroaryl, -O-C2∼C6 heterocyclyl, -OCH₂-C2∼C6 heterocyclyl, C1∼C6 ester, -NR₁₁R₁₁ₐ and 3-14 membered heterocyclyl containing 1-4 heteroatoms selected from the group consisting of N, O, S, SO and SO₂;
alternatively, R₁ and R₂ together form 3-14 membered heterocyclyl containing 1-4 heteroatoms selected from the group consisting of N, O, S, SO and SO₂, C3-C6 cycloalkyl, C6∼C12 aryl or C2∼C12 heteroaryl fused to a benzene ring;
R₇, R₈, R₉ and R₁₀ are independently selected from the group consisting of hydrogen, hydroxyl, C1∼C6 alkyl, C1∼C6 alkoxy and C1∼C6 alkylthio; and
R₁₁ and R₁₁ₐ are independently selected from the group consisting of a hydrogen atom or C1∼C6 alkyl.

3. The glycoside derivative according to claim 1 or 2, wherein A is an oxygen atom or -CH₂-;
B is an oxygen atom;
R₁, R₂, R₃, R₄, R₅ and R₆ are independently selected from the group consisting of hydrogen, hydroxyl, carboxyl, fluorine, chlorine, bromine, -CN, methyl, ethyl, n-propyl, isopropyl, n-butyl, isobutyl, tert-butyl, n-pentyl, isopentyl, n-hexyl, isohexyl, methoxy, ethoxy, n-propyloxy, isopropyloxy, n-butyloxy, isobutyloxy, tert-butyloxy, n-pentyloxy, isopentyloxy, n-hexyloxy, methoxymethoxy, ethoxymethoxy, ethoxyethoxy, n-propoxymethoxy, isopropoxymethoxy, n-propoxyethoxy, isopropoxyethoxy, cyclopropyl, methylcyclopropyl, cyclobutyl, cyclopentyl, cyclohexyl, phenyl, pyridyl, pyrimidyl, pyrazinyl, pyrrolyl, thienyl, furyl, oxazolyl, thiazolyl, imidazolyl, triazolyl, phenoxy, pyridyloxy, pyrimidinyloxy, pyrrolyloxy, furyloxy, thienyloxy, oxazolyloxy, benzyloxy, -OCH₂-pyridyl, -OCH₂-pyrimidinyl, -OCH₂-pyrrolyl, -OCH₂-thienyl, -OCH₂-furyl, -OCH₂-oxazolyl, -OCH₂-thiazolyl, -OCH₂-imidazolyl, -OCH₂-triazolyl, methyl ester, ethyl ester, isopropyl ester, sulfonate, amino, hexahydropyridyl, hexahydropyrazinyl, morpholinyl, tetrahydropyrrolyl and tetrahydrooxazolyl; and
the above groups are unsubstituted or one or more hydrogen atoms of the above groups are further substituted by one or more substituents selected from the group consisting of halogen, hydroxyl, amino, carboxyl, cyano, C1-C6 alkyl, C1-C6 alkoxy and nitro.

4. The glycoside derivative according to any one of claims 1-3, wherein R₁ and R₂ together form cyclohexyl, phenyl, pyridyl, pyrimidyl, pyrazinyl, pyrrolyl, thienyl, furyl, oxazolyl, thiazolyl, imidazolyl, triazolyl, hexahydropyridyl, hexahydropyrazinyl, morpholinyl, tetrahydropyrrolyl, tetrahydrooxazolyl or dioxane fused to a benzene ring.

5. The glycoside derivative according to any one of claims 1-4, wherein R₇, R₈, R₉ and R₁₀ are independently selected from the group consisting of hydrogen, hydroxyl, methyl, ethyl, n-propyl, isopropyl, n-butyl, isobutyl, tert-butyl, n-pentyl, isopentyl, n-hexyl, isohexyl, hydroxymethyl, hydroxyethyl, methoxy, ethoxy, n-propyloxy, isopropyloxy, n-butyloxy, isobutyloxy, tert-butyloxy, n-pentyloxy, isopentyloxy, n-hexyloxy, methylthio, ethylthio, n-propylthio, isopropylthio, n-butylthio, isobutylthio, tert-butylthio, n-pentylthio, isopentylthio and n-hexylthio.

6. The glycoside derivative according to any one of claims 1-5, which is a compound represented by formula II or a pharmaceutically acceptable salt thereof: 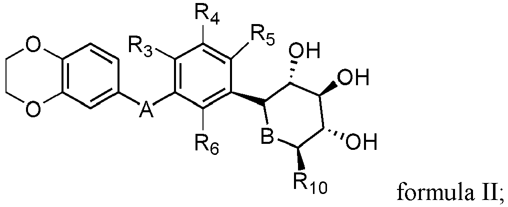
wherein, A is an oxygen atom, -(CH₂)ₘ- or -NH-; m is 1, 2 or 3;
B is an oxygen atom or a sulfur atom;
R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
R₁₀ is selected from the group consisting of hydrogen, hydroxyl, alkyl, alkoxy and alkylthio; and
R₁₁ and R₁₁ₐ are independently selected from the group consisting of a hydrogen atom and alkyl;
wherein, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio are unsubstituted or further substituted by one or more substituents selected from the group consisting of halogen, hydroxyl, amino, carboxyl, cyano, alkyl, alkoxy and nitro.

7. The glycoside derivative according to claim 6, wherein R₃, R₄, R₅ and R₆ are independently selected from the group consisting of hydrogen, hydroxyl, carboxyl, fluorine, chlorine, bromine, -CN, C1∼C6 alkyl, C1∼C6 alkoxy and C2∼C16 alkoxyalkoxy; and
R₁₀ is selected from the group consisting of hydrogen, hydroxyl, C1∼C6 alkyl, C1∼C6 alkoxy and C1∼C6 alkylthio.

8. The glycoside derivative according to claim 6 or 7, wherein R₃ is selected from the group consisting of C1∼C3 alkyl and C1∼C3 alkoxy;
R₄, R₅ and R₆ are hydrogen;
R₁₀ is selected from the group consisting of hydrogen, hydroxyl, C1∼C3 alkyl, C1∼C3 alkoxy and C1∼C3 alkylthio; and
the C1∼C3 alkyl, C1∼C3 alkoxy or C1∼C3 alkylthio is unsubstituted or further substituted by one or more substituents selected from the group consisting of halogen, hydroxyl, amino, carboxyl, cyano and nitro.

9. The glycoside derivative according to any one of claims 1 to 7, wherein,
A is an oxygen atom or -(CH₂)-;
B is an oxygen atom;
R₁ and R₂ together form 5 to 6-membered heterocyclyl fused to a benzene ring or form cyclobutanyl fused to a benzene ring, wherein the 5 to 6-membered heterocyclyl contains 1 or 2 heteroatoms selected from the group consisting of O and S;
R₃ is selected from the group consisting of CH₃(CH₂)ₙ-, CH₃(CH₂)ₙO- and halogen, wherein n is selected from 0, 1, 2 and 3;
R₄ is hydrogen;
alternatively, R₃ and R₄ together form cyclopentyl or oxacyclopentyl fused to a benzene ring;
R₅ and R₆ are hydrogen;
R₇, R₈ and R₉ are hydroxyl; and
R₁₀ is selected from the group consisting of hydroxymethyl, methoxy and methylthio.

10. The glycoside derivative according to claim 9, wherein,
A is an oxygen atom or -(CH₂)-;
B is an oxygen atom;
R₁ and R₂ together form 5 to 6-membered heterocyclyl fused to a benzene ring or form cyclobutanyl fused to a benzene ring, wherein the 5 to 6-membered heterocyclyl contains 1 or 2 oxygen atoms;
R₃ is selected from the group consisting of CH₃(CH₂)ₙ-, CH₃(CH₂)ₙO- and halogen, wherein n is selected from 0, 1, 2 and 3;
R₄ is hydrogen;
alternatively, R₃ and R₄ together form cyclopentyl or oxacyclopentyl fused to a benzene ring;
R₅ and R₆ are hydrogen;
R₇, R₈ and R₉ are hydroxyl; and
R₁₀ is selected from the group consisting of hydroxymethyl, methoxy and methylthio.

11. The glycoside derivative according to any one of claims 1 to 7, wherein,
A is an oxygen atom;
B is an oxygen atom;
R₁ is C₁-C₃ alkoxy;
R₂ is hydrogen;
R₃ is halogen, preferably fluorine, chlorine or bromine;
R₄ is hydrogen;
R₅ and R₆ are hydrogen;
R₇, R₈ and R₉ are hydroxyl; and
R₁₀ is hydroxyalkyl, preferably hydroxymethyl.

12. The glycoside derivative according to any one of claims 1-11, which has a structure selected from the group consisting of:

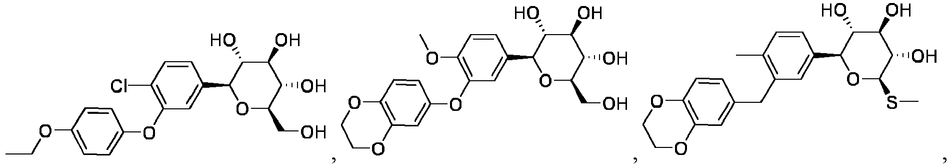

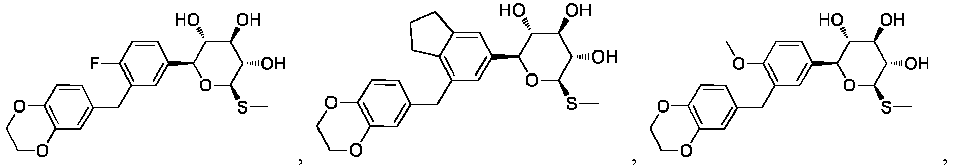

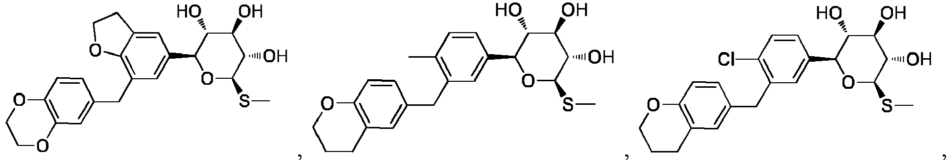

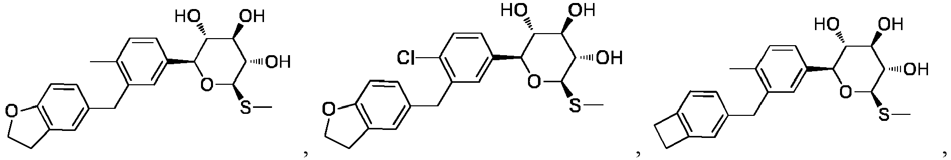

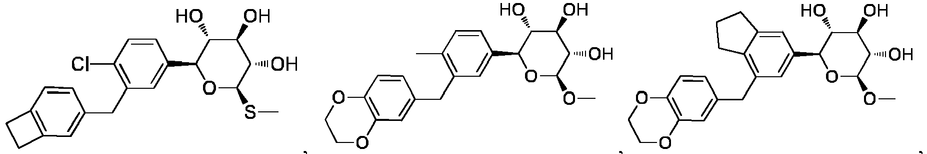

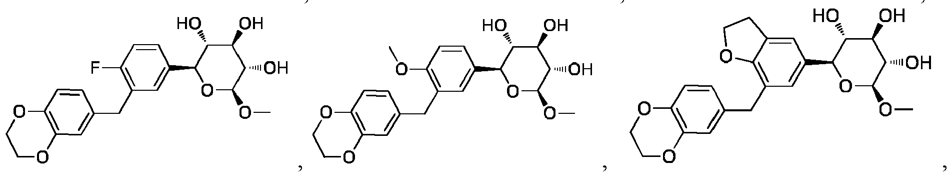

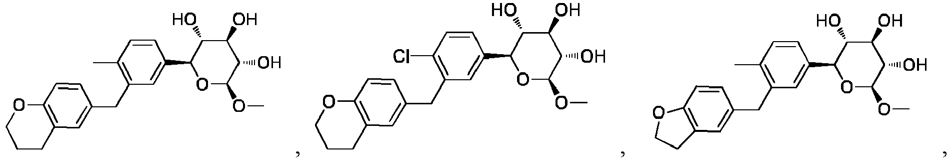

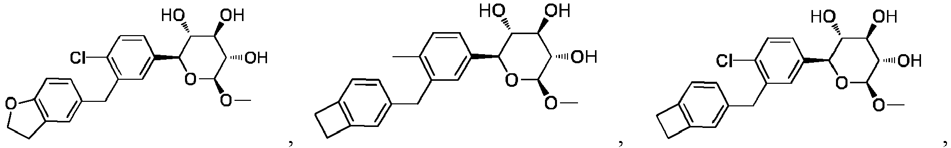

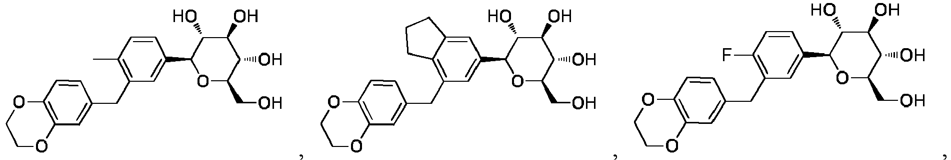

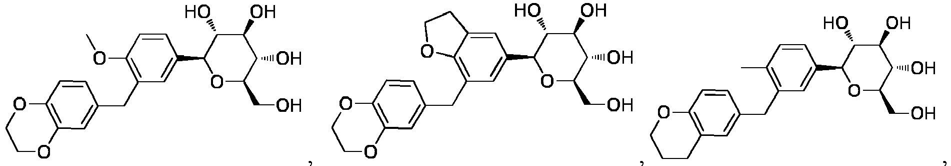

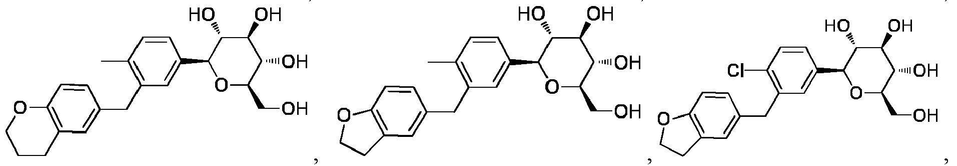

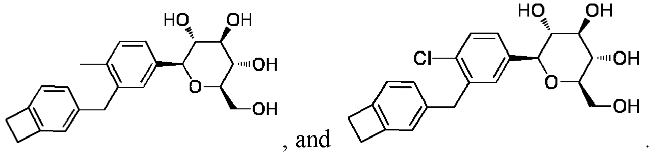

13. A method for producing the glycoside derivative according to any one of claims 1-12, comprising:
subjecting a compound represented by formula III to a deprotection reaction to prepare a compound represented by formula I-a; 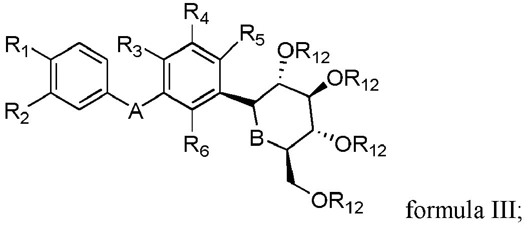 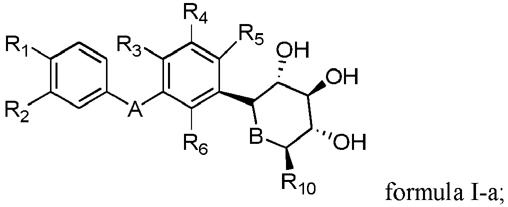
wherein, A is an oxygen atom, -(CH₂)ₘ- or -NH-; m is 1, 2 or 3;
B is an oxygen atom or a sulfur atom;
R₁, R₂, R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
alternatively, R₁ and R₂ together form heterocyclyl, cycloalkyl, aryl or heteroaryl fused to a benzene ring;
R₁₀ is hydroxymethyl; and
R₁₁ and R₁₁ₐ are independently selected from the group consisting of a hydrogen atom and alkyl;
wherein the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio are unsubstituted or further substituted by one or more substituents selected from the group consisting of halogen, hydroxyl, amino, carboxyl, cyano, alkyl, alkoxy and nitro; and
R₁₂ is selected from the group consisting of alkyl, trimethylsilyl, benzyl, formyl, acetyl, tetrahydropyranyl, methoxymethyl and tert-butyldimethylsilyl.

14. A method for producing the glycoside derivative according to any one of claims 1-12, comprising:
subjecting a compound represented by formula VII to sulfurization reaction and deprotection reaction to prepare a compound represented by formula I-a; 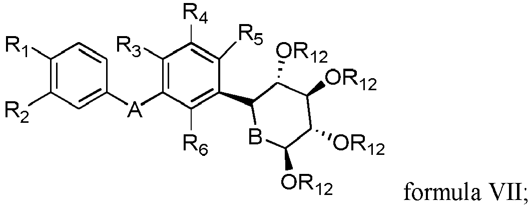 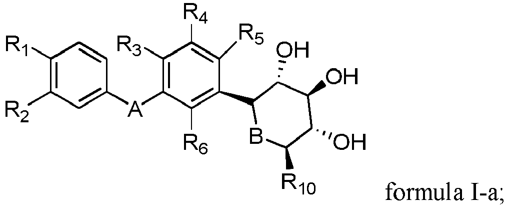
wherein, A is an oxygen atom, -(CH₂)ₘ- or -NH-; m is 1, 2 or 3;
B is an oxygen atom or a sulfur atom;
R₁, R₂, R₃, R₄, R₅ and R₆ are independently selected from the group consisting of hydrogen, hydroxyl, carboxyl, halogen, -CN, alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ and 3-14 membered heterocyclyl containing 1-4 heteroatoms selected from the group consisting of N, O, S, SO and SO₂;
alternatively, R₁ and R₂ together form heterocyclyl, cycloalkyl, aryl or heteroaryl fused to a benzene ring;
R₁₀ is methylthio; and
R₁₁ and R₁₁ₐ are independently selected from the group consisting of a hydrogen atom and alkyl;
wherein, the alkyl, alkoxy, alkoxyalkoxy, cycloalkyl, aryl, heteroaryl, -O-aryl, -O-heteroaryl, -OCH₂-aryl, -OCH₂-heteroaryl, -O-heterocyclyl, -OCH₂-heterocyclyl, ester, -NR₁₁R₁₁ₐ, heterocyclyl and alkylthio are unsubstituted or further substituted by one or more substituents selected from the group consisting of halogen, hydroxyl, amino, carboxyl, cyano, alkyl, alkoxy and nitro; and
R₁₂ is selected from the group consisting of alkyl, trimethylsilyl, benzyl, formyl, acetyl, tetrahydropyranyl, methoxymethyl and tert-butyldimethylsilyl.

15. A pharmaceutical composition, comprising the glycoside derivative according to any one of claims 1-12 or the glycoside derivative produced by the method according to claim 13 or 14, and a pharmaceutically acceptable carrier, excipient, diluent, adjuvant, vehicle or a combination thereof.

16. Use of the glycoside derivative according to any one of claims 1-12, the glycoside derivative produced by the method according to claim 13 or 14, or the pharmaceutical composition according to claim 15 in the manufacture of a medicament for preventing, treating or alleviating diabetes mellitus and a complication thereof.

17. The use according to claim 16, wherein the diabetes mellitus is type II diabetes mellitus or type I diabetes mellitus; and the complication is a cardiovascular disease caused by type II or type I diabetes mellitus.
